# EUROPEAN PATENT SPECIFICATION

(11) **EP 1 283 716 B1**
(45) Date of publication and mention of the grant of the patent: **28.03.2012**
(21) Application number: 01923827.8
(22) Date of filing: 25.04.2001
(51) Int. Cl.: A61K 39/00, C07K 14/435, C07K 16/18, C12N 15/12, A61P 33/14

(54) **VACCINE COMPRISING A TICK CEMENT PROTEIN**
ZECKENZEMENTPROTEIN-ENTHALTENDE IMPFSTOFF
VACCIN CONTENANT UNE PROTEINE DE CEMENT DE TIQUE

(30) Priority: 25.04.2000 GB 0010068; 23.11.2000 GB 0028606
(43) Date of publication of application: 19.02.2003
(73) Proprietor: Merial Limited, Harlow, Essex CM19 5QA (GB)
(72) Inventor: Trimnell, Adama Roseanne, Headington, Oxford OX3 9NS (GB); Paesen, Guido Christiaan, Jericho, Oxford OX2 6DB (GB); Nuttall, Patricia Anne, Culham, Oxon OX14 4NP (GB)
(74) Representative: Harding, Charles Thomas
(86) International application number: PCT/GB2001/001834
(87) International publication number: WO 2001/080881

(56) References cited:
- WO-A-01/40469
- WO-A-99/24567
- S. SHAPIRO ET AL.: "Acquired resistance to ixodid ticks induced by tick cement antigen" EXP. APPL. ACAROL., vol. 7, 1989, pages 33-41, XP002093750
- A. MULENGA ET AL.: "Molecular characterisation of a H. longicornus tick salivary gland associated 29kd protein and its effect as a vaccine against tick inffestation in rabbits" INF. IMMUN., vol. 67, no. 4, 1999, pages 1652-1658, XP002175122
- S. BROWN: "A. americanum: physiochemical isolation of a protein derived from tick salivary gland that is capable of inducing immune resistance in guinea pigs" EXP. PARASITOL., vol. 62, 1986, pages 40-50, XP002093752
- D. JAWORSKI ET AL.: "Tick (Acari:Ixodidae) attachment cement and salivary gland cells contain similar immunoreactive peptides" J. MED. ENTOMOL., vol. 29, no. 2, 1992, pages 305-309, XP002093749

## Description

The present description relates to the use of a tick cement protein in the production of vaccines for protecting animals against the bite of blood-sucking ectoparasites and against the transmission of viruses, bacteria and other pathogens by such ectoparasites.

Blood-sucking ectoparasites, such as mosquitoes and ticks, are extremely effective as transmitters of disease. For example, the tick species *R. appendiculatus* represents a major obstacle to livestock development in several sub-saharan regions, transmitting the protozoan parasite *Theileria parva* which causes the usually fatal East Coast Fever. This disease is often considered the most important disease of cattle (Norval *et al.,* 1992a; Norval *et al.,* 1992b). This tick is also the main vector of the virus causing Nairobi sheep disease, a disabling and often deadly disease in sheep and goats (Davies, 1988). Furthermore, R *appendiculatus* and other tick pests also cause considerable damage to animals' skin, thereby affecting the leather industry.

Conventionally, techniques to control tick populations have used the treatment of animals with chemicals such as acaracides. This strategy has resulted in the development of resistant ticks, meaning that new classes of chemicals must be introduced Furthermore, the chemicals have little residual effect, meaning that they must be applied frequently. A second approach is to breed for tick-resistant animals, but the degree of resistance that results is far from ideal.

In an effort to combat parasite-transmitted diseases, a number of attempts have been made to immunise animals against ticks using extracts of whole ticks or of tick gut. Certain reports have used recombinant tick proteins (see, for example, International patent application WO88/03929). However, despite such developments, the only commercially-available tick vaccines are active only against the adult stage of *B. microplus* ticks and show variation in efficacy depending on the geographical location of this species.

No vaccines have yet been developed that provide resistance across entire populations of vaccinated animals or against parasites at every stage of their life cycle. There therefore exists a great need for an effective vaccine to combat diseases that are transmitted by blood-feeding ectoparasites. Surprisingly, it has now been discovered that tick cement proteins are useful as vaccine components.

### Summary of the invention

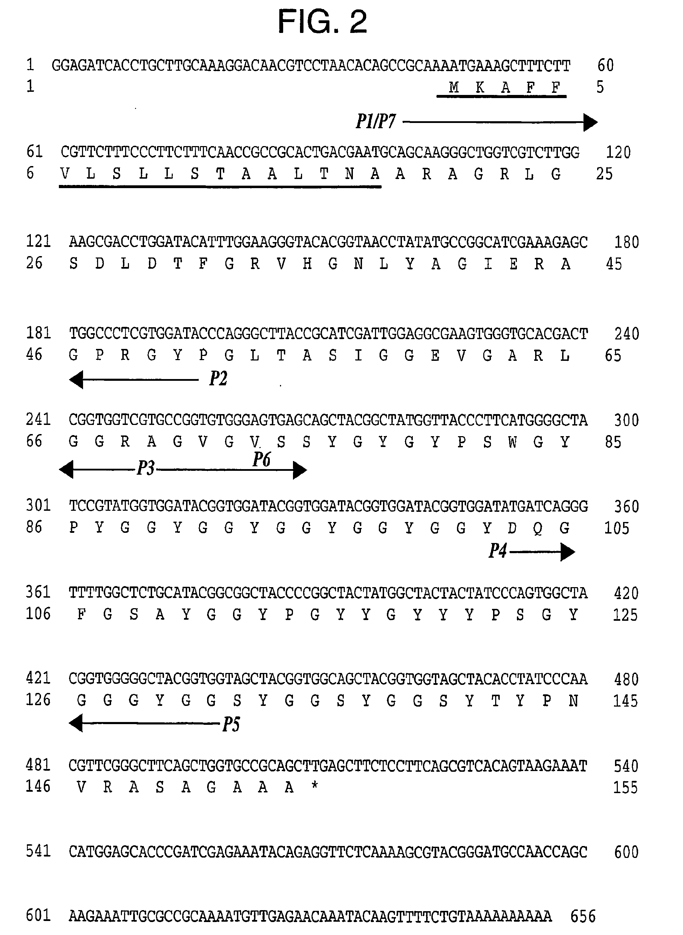

The present invention relates to a vaccine composition comprising a recombinant fragment of the 64P cement protein of the tick *Rhipicephalus appendiculatus* an active component in conjunction with a pharmaceutically acceptable excipient, wherein said fragment is selected from the group consisting of:
64trp2 (an N-terminal fragment of the 64P tick cement protein consisting of 51 amino acids cloned as a glutathione-s-transferase and histidine tag fusion protein wherein said 51 amino acids are amino acids 1-51 of the amino acid sequence in Figure 2);
64trp3 (an N-terminal fragment of the 64P tick cement protein consisting of 70 amino acids cloned as a glutathione-s-transferase and histidine tag fusion protein wherein said 70 amino acids are amino acids 1-70 of the amino acid sequence in Figure 2);
64trp5 (a fragment of the 64P tick cement protein consisting of 133 amino acids cloned as a glutathione-s-transferase fusion protein wherein said 133 amino acids are amino acids 1-133 of the amino acid sequence in Figure 2);
64trp6 (a fragment of the 64P tick cement protein consisting of 133 amino acids cloned as a glutathione-s-transferase and histidine tag fusion protein wherein said 133 amino acids are amino acids 1-133 of the amino acid sequence in Figure 2); and combinations thereof.

An antibody or an antiserum that is reactive with a tick cement protein fragment as defined herein, are also disclosed.

In a further aspect, the present invention relates to a non-therapeutic and non-surgical method of production of an antibody or an antiserum according to the present invention, comprising immunising an animal with a vaccine composition according to the present invention.

The present invention, in a further aspect, relates to the use of a vaccine composition according to the present invention in the preparation of a medicament for immunising a mammal against a tick.

The present invention, in another aspect, relates to a vaccine composition according to the present invention for use in immunising a mammal against a tick.

According to the present description there is provided a vaccine composition comprising an immunogenic tick cement protein, a fragment thereof or a functional equivalent thereof, in conjunction with a pharmaceutically-acceptable excipient. Immunisation of an animal with such a vaccine is shown herein to cause the generation of antibodies that are effective against a wide variety of ectoparasite species.

A large number of ectoparasite species exist in various parts of the world, although their incidence tends to be concentrated in tropical and sub-tropical regions, where they, and diseases carried by them, are endemic. These species vary greatly in type and adopt widely differing feeding strategies, ranging from transient feeders such as mosquitoes, horseflies, tsetse flies, fleas, lice and mites, down to leeches and ticks, some of which may feed for long periods of time. All of these ectoparasite species are suitable targets for the vaccines of the description.

The vaccines of the description are particularly efficacious against tick species. Examples of such targeted tick species are *Rhipicephalus appendiculatus, R. sanguineus, R. bursa, Amblyomma variegatum, A. americanum, A. cajennense, A. hebraeum, Boophilus microplus, B. annulatus, B. decoloratus, Dermacentor reticulatus, D. andersoni, D. marginazus, D. variabilis, Haemaphysalis inermis, Ha. leachii, Ha. punctata, Hyalomma anatolicum anatolicum, Hy. dromedarii, Hy. marginatum marginatum, Ixodes ricinus, I. persulcatus, I. scapularis, I. hexagonus, Argas persicus, A. reflexus, Ornithodoros erraticus, O. moubata moubata, O. m. porcinis, and O. savignyi.*

What is common between all the ectoparasite species mentioned above is that they ingest either blood, lymph or they feed on host skin products, meaning that any antibodies present in their host are automatically internalised into the ectoparasite. This provides an advantageous and automatic route of administration for antibody and, provided that the antibody is reactive against an ectoparasite protein, means that a well-organized immunisation regime can result in the complete eradication of the parasite within the area concerned. Ectoparasites that feed on blood are particularly preferred targets for the vaccines of the description.

The inventors have discovered a number of different tick cement proteins and, in many cases, have cloned their encoding genes. For example, International patent application WO 9924567 describes the isolation of number of tissue cement proteins, and discusses their use in medicine as components of tissue cement for use in skin surgery and wound healing, and for the temporary or permanent bonding of human or animal tissues to each other or to other biomaterials.

Ixodid (hard) ticks are haematophagous parasites that attach themselves to a vertebrate host by means of a 'cement cone', originating from the type II and type III acini of the tick salivary glands (Kemp *et al.,* 1982; Walker *et al.,* 1985).

The cement that forms the cone is a milky-white secretion that is injected into the skin of animals on which these parasites feed. The cement comprises a number of interacting protein and carbohydrate components. The cement spreads into the bite site and over the skin and, upon hardening, ensures that the mouthparts remain firmly anchored to the host during the feeding period, which typically lasts 4 to 8 days. The cement cone functions additionally as a gasket to prevent leakage of fluids from the bite site during feeding.

The tick cement cone is a layered structure, constructed from two major types of cement. The first type of cement is produced just minutes after establishing the bite site and hardens quickly to form a rigid 'core' of the cone. A second type of cement is secreted later, about 24 hours after attachment, and hardens more slowly to form a more flexible 'cortex'. In adult ticks, cement production typically continues until the 3rd or 4th day after attachment (Kemp *et al.,* 1982; Sonnenshine *et al.,* 1991).

The composition of tick cement appears to be similar amongst different Ixodid tick species. For example, an antiserum raised against a 90kD salivary protein of the brown ear tick, *Rhipicephalus appendiculatus*, has been shown to recognise polypeptides from the salivary glands and cement proteins of the American dog tick, *Dermacenter variabilis*, the lone star tick, *Amblyomma americanum,* and the brown dog tick, *R. sanguineus* (Jaworski *et al.,* 1992).

Unpurified cement components have previously been tested as inducers of host resistance (Brown *et al.,* 1986; Shapiro *et al.,* 1989), but reliable vaccines based on these proteins have not been developed. In a sense, this is not surprising, because the sequence relationships indicate that the cement protein has been designed to resemble the skin proteins of the host, with the most likely aim of avoiding rejection of skin to tick attachment by the host's natural immune defence mechanisms. The compositional resemblance of certain tick proteins with their surrounding tissues may also facilitate the intimate binding between the cement cone and the surrounding skin tissues.

Tick cement proteins suitable for incorporation in the vaccines of the description may be derived from any suitable tick species, such as the species *Rhipicephalus appendiculatus, I. ricinus, Dermacenter reticulatus, Dermacenter variabilis, Amblyomma americanum, Rhipicephalus sanguineus, Amblyomma variegatum, Boophilus microplus,* and *Haemaphysalis leachii.* In a preferred embodiment, the tick cement protein is derived from the tick *R. appendiculatus.*

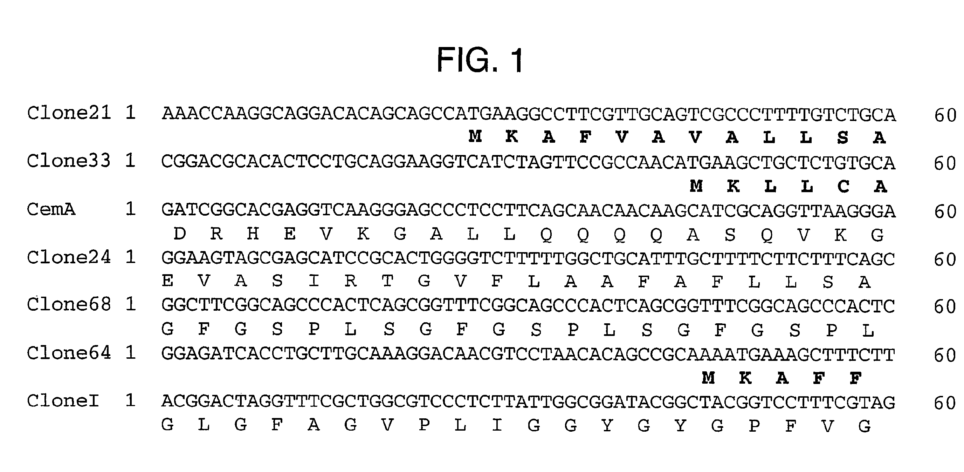

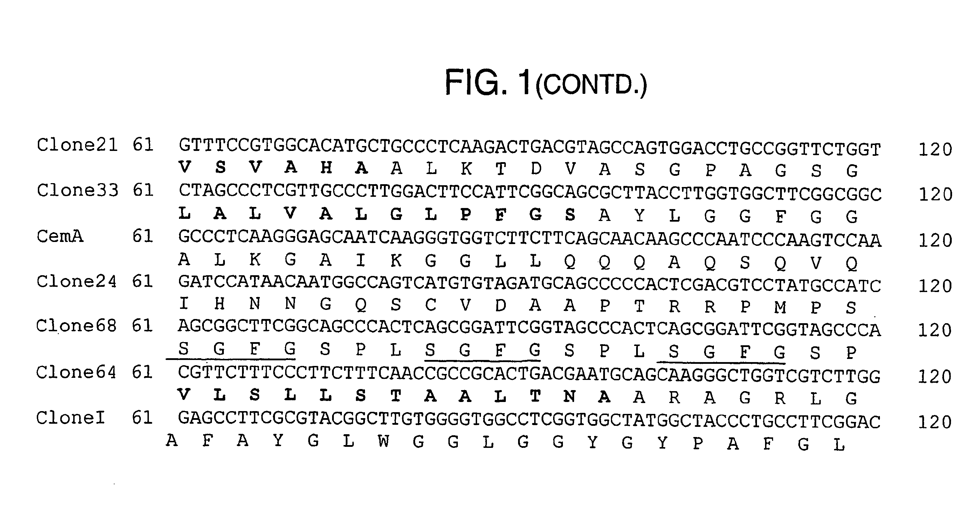

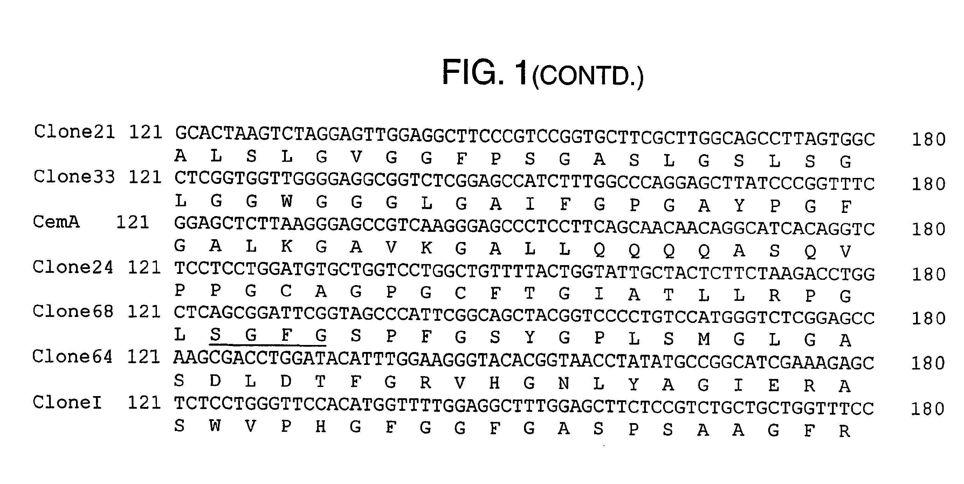

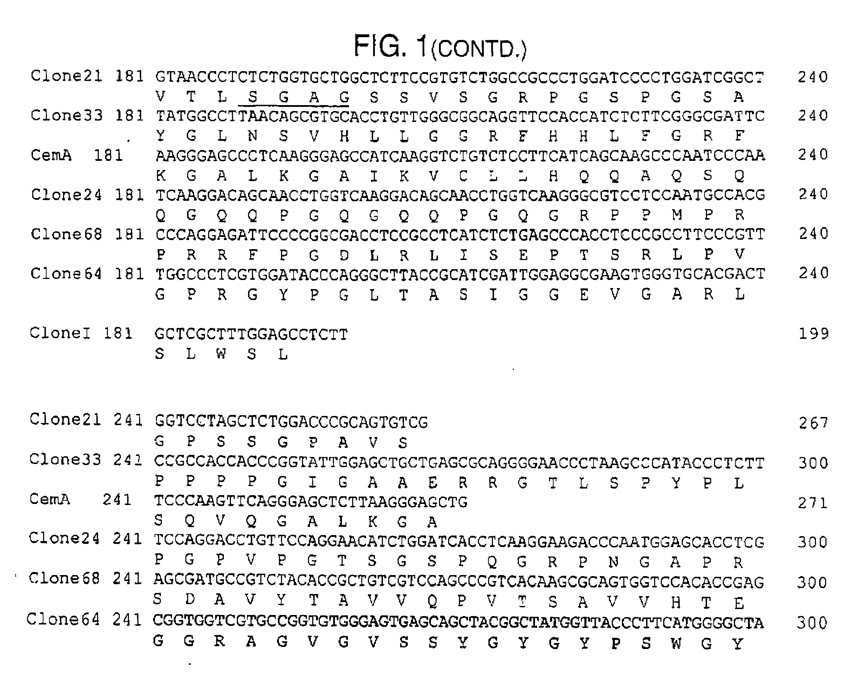

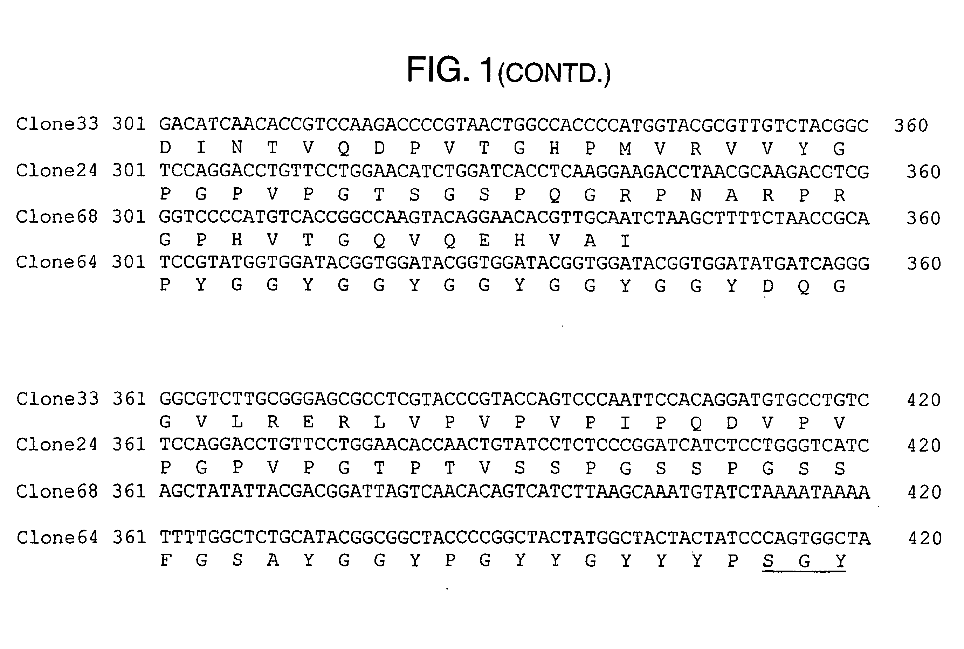

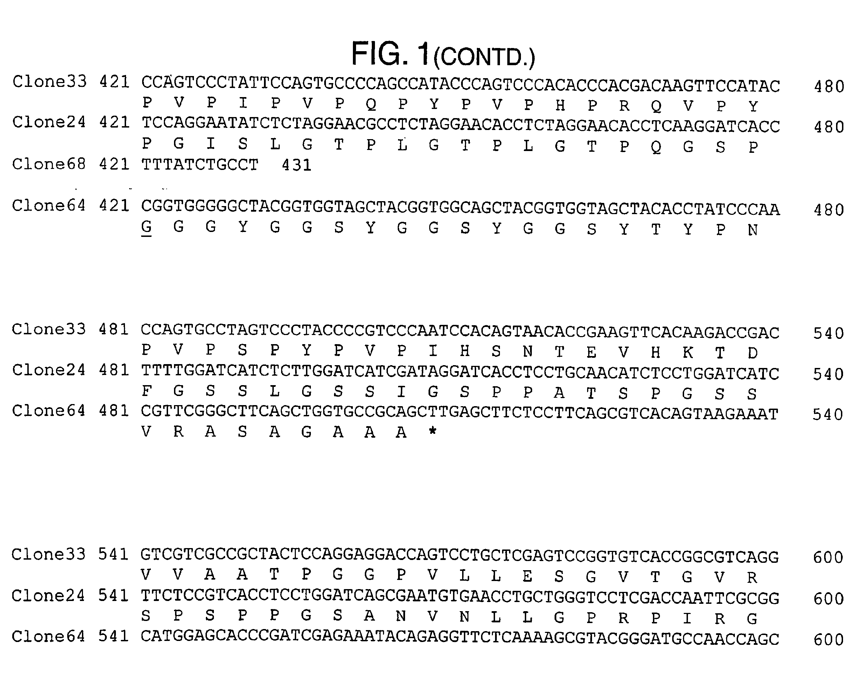

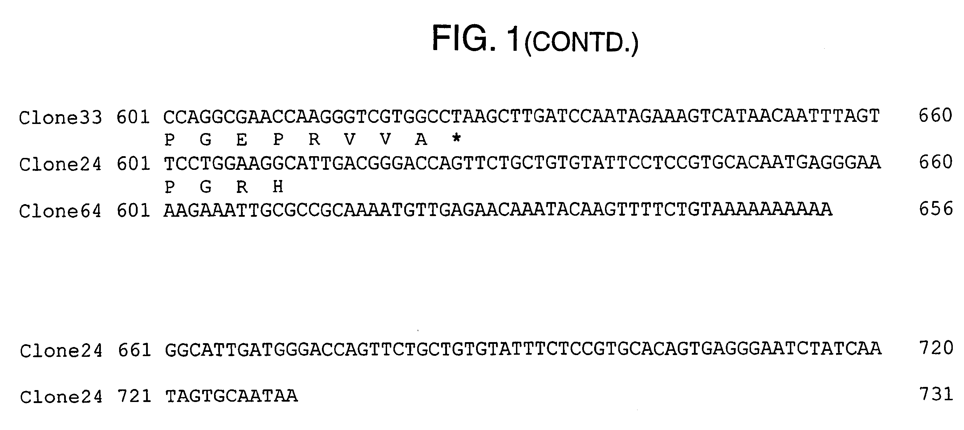

Examples of particularly suitable tick cement proteins for inclusion in the vaccines of the description are given herein and also in patent application WO 9924567. These cement proteins include proteins referred to as clone 21, clone 33, CemA, clone 24, clone 68, clone 64 and clone I. The predicted amino acid sequences of these proteins are identified in Figure 1 herein.

Preferably, the tick cement protein, fragment thereof or functional equivalent thereof used in the vaccine composition of the description should contain an immunogenic epitope that is present in one or more orthologous proteins of a blood-feeding ectoparasite species other than the tick species from which said immunogenic cement protein, fragment or functional equivalent is derived. This will mean that one single vaccine composition may be effective as a broad spectrum vaccine against all of the ectoparasite species that produce a protein containing the common epitope. For example, in certain areas, a number of different tick species are endemic and cause a significant pest problem for the farming industry. The availability of a single vaccine that is effective against a number of different tick species will reduce the cost of administering the vaccine and will be thus be advantageous over currently available vaccines.

The vaccines of this aspect of the description are thus particularly advantageous because an inflammatory response is stimulated that will boost the immune status of vaccinated animals and, in addition, will target concealed antigens, so resulting in damage to the tick itself.

Certain tick cement proteins, and fragments of these proteins, have been discovered, according to one aspect of the present description, to contain epitopes that also exist in proteins that are present in the gut and haemolymph of ectoparasite species. This cross-reactivity makes the vaccines of this embodiment of the description particularly advantageous, since ingestion of blood, and thus host antibodies, into the ectoparasite guarantees delivery of the active agent to the parasite. In this manner, the vaccines of the description target species that feed transitorily, such as mosquitoes and horseflies, as efficiently as those species that remain attached to their host for a significant period of time, such as ticks.

A particularly preferred protein for inclusion in a vaccine according to the description is the clone 64 protein (hereafter 64P), a fragment thereof, or a functional equivalent thereof, for example, isolated from a species other than *R. appendiculatus.* This protein possesses a sequence typical of a structural protein, and appears to be secreted in the saliva of ticks. The sequence comprising the first 40 amino acids of the cement protein is strongly collagen-like, whereas the rest of the sequence resembles keratin. Homology searches conducted with the sequence of this protein reveals that the highest level of homology for all searched sequences in the Genbank database (http://www.ncbi.nlm.nih.gov) was 51%, for mouse epidermal keratin subunit I.

The protein is glycine-rich and contains several repeats of the motif (C/S) 1-4 (Y/F), resembling structural proteins from *Drosophila melanogaster* (cuticular protein) and other insect egg shells, as well as vertebrate cytokeratins including mammalian keratin complex 2 basic protein, mouse keratin, human keratin, collagen type IV alpha, and IP1B2 precursor.

In one embodiment of the description functional equivalents of tick proteins may be included in the vaccine compositions. The term "functional equivalent" is used herein to describe those proteins that have an analogous function to the cement proteins termed clone 21, clone 33, CemA, clone 24, clone 68, clone 64 and clone I. Functionally-equivalent proteins may belong to the same protein family as these proteins. By protein family is meant a group of polypeptides that share a common function and exhibit common sequence homology between motifs that are present in the polypeptide sequences.

By "sequence homology" is meant that the polypeptide sequences are related by divergence from a common ancestor. In particular, the proteins and partial proteins identified herein possess certain sequences in common that are repeated several times throughout the sequence of the protein. Preferably, the homology between polypeptide sequences in the same protein family is at least 30% across the whole of the amino acid sequence of the protein. More preferably, the homology is at least 50%, at least 60%, or at least 70% across the whole of the amino acid sequence of the protein. Even more preferably, homology is greater than 80%, 95%, 90%, 95%, 96%, 97%, 98% or 99% across the whole of the protein sequence.

By "analogous function" is meant firstly that the proteins have retained the capacity to form a cement, at least when present with other cement proteins. In combination with other necessary cement constituents, such proteins will thus be capable of hardening over a period of time to form a solid mass or glue. Secondly, this term may refer to proteins that are structurally similar to cement proteins and that thus contain similar or identical epitopes.

Functional equivalents of tissue cement proteins include mutants containing amino acid substitutions, insertions or deletions from the wild type sequence, provided that immunogenicity is retained. Functional equivalents with improved immunogenicity from that of the wild type protein sequence may also be designed through the systematic or directed mutation of specific residues in the protein sequence.

Functional equivalents include proteins containing conservative amino acid substitutions that do not affect the function or activity of the protein in an adverse manner. This term is also intended to include natural biological variants (e.g. allelic variants or geographical variations within the species from which the tissue cement proteins are derived).

According to the description fragments of tick cement proteins are also envisioned as suitable components for inclusion in the vaccine compositions. For example, short stretches of peptide derived from immunogenic portions of tick cement proteins may be particularly useful as immunogens. Such short stretches of polypeptide sequence are simple to produce in large quantities, either synthetically or through recombinant means. Protein fragments may in many instances be preferred for use in the vaccines of the description since these fragments are likely to fold into conformations not adopted by the full length wild type sequence. Since some cement proteins are likely to have evolved so as to resemble the tissues of the host skin and thus to avoid provoking a host immune response against the tick, such unnatural forms of tick cement proteins are likely to be of particular use in the vaccines of the present description. Examples of fragments of tick cement proteins useful for inclusion in the vaccine compositions of the description include various fragments of the 64P protein that have been generated recombinantly by the inventors, and functional equivalents of these-fragments, such as close homologues and mutants of the kind discussed above. As will be apparent to the skilled reader, similar fragments to those that are explicitly disclosed herein may be prepared from ectoparasite species other than the tick species *R. appendiculatus.*

The details of the *R. appendiculatus* fragments described herein are as follows.

The fragment termed 64trp1 is a small C-terminal fragment of the 64P protein consisting of 29 amino acids cloned as a glutathione-s-transferase (GST / histidine tag fusion protein with a molecular weight of around 30 kDa.

The fragment termed 64trp2 refers to a small N-terminal fragment of the 64P protein consisting of 51 amino acids cloned as a glutathione-s-transferase (GST) / histidine tag fusion protein with a molecular weight of around 33 kDa.

The fragment termed 64trp3 refers to a larger N-terminal fragment of 64P protein consisting of 70 amino acids cloned as a glutathione-s-transferase (GST) / histidine tag fusion protein with a molecular weight of around 36 kDa.

The fragment termed 64trp6 refers to the full-length clone of 64P protein consisting of 133 amino acids cloned as a glutathione-s-transferase (GST) / histidine tag fusion protein. This fragment has an approximate molecular weight of around 42 kDa.

The fragment termed 64trp4 is a C-terminal fragment of 64P protein consisting of 63 amino acids cloned as a glutathione-s-transferase (GST) / histidine tag fusion protein with a molecular weight of around 35 kDa.

The fragment termed 64trp5 is the full-length clone of 64P protein sequence consisting of 133 amino acids cloned as a GST fusion protein (i.e. minus the histidine tag). This protein has a molecular weight of 41 kDa.

These protein fragments, and functional equivalents thereof, are particularly preferred components for incorporation in the vaccines of the description. These fragments may be expressed as soluble protein, or may alternatively be expressed in inclusion bodies and purified under denaturing conditions. For example, the construct 64trp6 as isolated from R. *appendiculatus* has been prepared as a denatured protein expressed in inclusion bodies and demonstrated to be immunogenic in this form.

Immunisation with these protein fragments, followed by attachment of ectoparasite, results in inflammation at the attachment site and subsequent death of the ectoparasite. The skilled reader will appreciate that the presence of the heterologous GST and HIS tag sequences is purely for convenience of protein production. These stretches of sequence are not considered to be essential to this aspect of the description.

Conveniently, the vaccines according to the description contain a tick cement protein, fragment thereof or functional equivalent thereof, expressed in recombinant form. Recombinantly-expressed protein is inexpensive to produce and, using the now standard techniques of genetic engineering, allows the simple manipulation of gene sequences to give a desired protein product.

It is preferred that the vaccines of the description are effective against both adult and immature forms of the ectoparasite. The term "immature" is meant to include both nymph and larval forms of the ectoparasite. This means that the whole ectoparasite population may be targeted using the vaccine, so increasing the efficiency of ectoparasite eradication.

The vaccines may specifically target adult or immature forms of ectoparasites, but will preferably target all parasitic stages of the life cycle. Of the fragments specifically exemplified herein, 64trp2-, 64trp3-, 64trp5- and 64trp6-immunised animals caused significant mortality in tick nymphs or adult ticks or both nymphs and adults, depending on the tick species, and these fragments are thus particularly preferred. A cocktail of 64trp2 + 64trp6 was effective against both adult and immature forms of tick ectoparasites; these particular fragments used in combination are thus particularly preferred for inclusion in a vaccine according to the invention.

According to a further embodiment of the invention, there is provided a cocktail vaccine comprising two or more tick cement proteins, fragments or functional equivalents, optionally in conjunction with an adjuvant Any two or more immunogenic tick cement proteins, protein fragments or functional equivalents may be used as components of such as cocktail vaccine, and may be from different or from the same tick species. For example, it may be desired to generate a vaccine that specifically targets more than one ectoparasite, or that targets different proteins from the same ectoparasite. In this manner, it may be possible to generate a more efficacious vaccine with greater species coverage. Particularly preferred combinations of components include the combination of 64trp2, 64trp3, 64trp5 and 64trp6, the.combination of 64trp2 and 64trp6 and the combination of 64trp3 and 64trp6. These combinations are demonstrated herein to possess particular efficacy in targeting both adult and immature ticks and in conferring cross-species resistance.

Vaccine compositions according to the description may also comprise additional agents, for example, molecules that the ectoparasite uses to promote pathogen transmission, such as interferon regulators, complement inhibitors, chemokine regulators and immunoglobulin-binding proteins. In this way, other bioactive molecules that are released from the salivary glands of ectoparasites may be recognised as foreign by the host immune system and an immune response mounted.

A further aspect of the present description comprises a vaccine containing a tick cement protein fused to another molecule, such as a label, a toxin or other bioactive or immunogenic molecule. Particularly suitable candidates for fusion may be a molecule such as glutathione-s-transferase or a histidine tag, although luciferase, green fluorescent protein or horse radish peroxidase may also be suitable. Linker molecules such as streptavidin or biotin may also be used, for example, to facilitate purification of the cement protein.

Fusion proteins may be created chemically, using methods such as chemical cross-linking. Such methods will be well known to those of skill in the art and may comprise, for example, cross-linking of the thiol groups of cysteine residues. Chemical cross-linking will in most instances be used to fuse tissue cement proteins to non-protein molecules, such as labels.

When it is desired to fuse a tissue cement protein to another protein molecule, the method of choice will generally be to fuse the molecules genetically. In order to generate.a recombinant fusion protein, the genes or gene portions that encode the proteins or protein fragments of interest are engineered so as to form one contiguous gene arranged so that the codons of the two gene sequences are transcribed in frame.

Immunisation with naked, plasmid DNA encoding specific antigens has recently been acknowledged as an efficient method of presenting antigens to the mammalian immune system, resulting in strong humoral and cellular immune responses (Ulmer et al., Science 1993, 259, 1745-1749). This technique, also referred to as DNA vaccination, has been successfully applied to generate antibodies directed against several proteins derived from viruses (Ulmer *et al.*, *loc cit.*; Cox et al., J. Virol. 1993, 67, 5664-5667; Fynan et al., Proc. Natl. Acad. Sci. USA 1993, 90, 11478-11482; Robinson et al., Vaccine 1993, 11, 957-960; Wang et al., 1993, DNA Cell Biol. 1993, 12, 799-805; Davis et al., Hum. Mol. Genet 1993, 2, 1847-1851; Xiang et al., Virology 1994, 199, 132-140; Xiang et al., Virology 1995, 209, 569-579; and Justewicz et al., J. Virol. 1995, 69, 7712-7717), parasites (Sedegah et al., Proc. Natl. Acad. Sci. USA 1994, 91, 9866-9870; Mor et al., J. Immunol. 1995, 155, 2039-2046; and Yang et al., Biochem. Bioph. Res. Comm. 1995, 212, 1029-1039) and bacteria (Anderson et al., Infect. Immun. 1996, 64, 3168-3173), and, in several cases, a significant protective response has been elicited by the host. These DNA vaccines continuously stimulate the immune system, amplifying immunity and thereby reducing the cost of production and delivery as no booster injections are required.

Based on the available evidence, immunisation with plasmid DNA encoding the various tick cement proteins is likely to be a useful technique to further improve their anti-tick vaccine effects. The method would involve direct injection of the host with a eukaryotic expression vector such that one or more cement proteins are expressed by in vivo transcription then translation of the corresponding sequence within the vaccinated host (humans, livestock, or other animals).

The vaccines of any one of the above-described aspects of the invention may additionally comprise an adjuvant. Suitable adjuvants to enhance the effectiveness of the immunogenic proteins according to the present description include, but are not limited to, oil-in-water emulsion formulations (optionally including other specific immunostimulating agents such as muramyl peptides or bacterial cell wall components), such as for example (a) those formulations described in PCT Publ. No. WO 90/14837. Other suitable adjuvants will be known to those of skill in the art and include Saponin adjuvants, such as Stimulon^{™} (Cambridge Bioscience, Worcester, MA), ISA Montanide 50, cytokines, such as interleukins, interferons, macrophage colony stimulating factor (M-CSF) or tumor necrosis factor (TNF).

According to a further embodiment of the description, there is provided a monoclonal antibody that is reactive with a tick cement protein. By "reactive is meant that the antibody binds to one or more tick epitopes with an affinity of at least 10⁻⁸M, preferably at least 10⁻⁹M, more preferably at least 10⁻¹⁰M. According to a preferred embodiment of this aspect of the description the antibody or antiserum is reactive against any one or more of the cement proteins, fragments or functional equivalents that are specifically recited above. This aspect of the description includes a method for the production of such an antibody or an antiserum, comprising immunising an animal with a tick cement protein, fragment thereof, or functional equivalent thereof as listed in any one of the above-described aspects of the description.

According to a still further aspect of the description, there is provided a process for the formulation of a vaccine composition comprising bringing a tick cement protein, a fragment or a functional equivalent into association with a pharmaceutically-acceptable carrier, optionally in conjunction with an adjuvant.

According to a still further aspect of the present description there is provided a method of immunising a mammal against an ectoparasite-transmitted disease or against a blood-feeding ectoparasite, comprising administering to an animal, a vaccine according to any one of the above-described aspects of the invention.

The description also provides a tick cement protein, fragment thereof or functional equivalent thereof, for use in a vaccine. The description further provides for the use of a tick cement protein as a component of a vaccine.

Various aspects and embodiments of the present description will now be described in more detail by way of example, with particular reference to tick cement proteins isolated from ticks, and especially from *Rhipicephalus appendiculatus.* It will be appreciated that modification of detail may be made without departing from the scope of the invention.

### Brief description of the Figures

Figure 1: Nucleotide and inferred amino acid sequences of seven clones of putative cement proteins.
   Clone 21: Partial cDNA sequence and translation product of clone 21. The cDNA-inferred protein is predicted to be a cement protein; it contains a hydrophobic N-terminal region which possibly constitutes a signal sequence, typical for secretion products, and it strongly resembles structural proteins, especially keratin. A recognition sequence for post-translational attachment of glycosaminoglycan groups is underlined.
   Clone 33: Inferred protein sequence of PCR-cloned DNA product (from cDNA library) into the fusion protein expression vector, pGEX-2T (Pharmacia). A putative signal sequence is given in bold. Like many structural proteins, this protein is glycine- and proline-rich. It has some resemblance with keratins. * indicates the stop codon.
   Clone cemA: Partial sequence of the cemA cDNA and protein (putative reading-frame). The protein is very repetitive, with the sequence KGALLQQQQASQVKGALKAI, or slight variants thereof, repeated several times.
   Clone 24: Incomplete cDNA and cDNA-inferred sequence of clone 24. The protein has resemblance to structural proteins (amongst others collagen), and contains repeat sequences. Many related clones are found in the library. The cDNA has also got a region in common with glutenin, a self-assembling protein.
   Clone 68: Partial cDNA and cDNA-inferred sequence of clone 68. The library contains a family of similar clones. The encoded proteins resemble structural proteins, such as keratin. A series of possible glycosaminoglycan attachment sites are underlined
   Clone 64: Complete cDNA sequence and cDNA-inferred protein sequence of clone 64. The putative signal sequence is given in bold. A possible glycosaminoglycan attachment site is underlined. The first 40 amino-acid piece of the mature protein is collagen-like, the remainder of the sequence resembles keratin. The protein is glycine-rich and contains several repeats of the motif (C/S)1-4(Y/F), which is also found in structural proteins from insect egg shells. The tyrosines may be involved in cross-linking by formation of dityrosine-bridges by phenoloxidases. A similar protein is encoded by clone I (see below). * indicates the stop codon.
   Clone I: Incomplete cDNA-sequence and cDNA-inferred protein sequence of clone I. The inferred protein is glycine- and tyrosine-rich and resembles a cement protein of the reef-building polychaete Pragmatopoma californica (a component of the quinone-tanned cement in the tubes built by these marine worms).
Figure 2: Amino acid sequences of 64P protein fragments (64TRPs) expressed in *Escherichia coli.* P1/ P2, P1/P3, P4/P5, P6/P5, P1/P5 and P7/P5 refer to primers used to subclone PCR products from 64P amino acid sequence into the plasmid pGEX-2T, for expression in *Escherichia coli* cells as truncated versions of 64P protein, i.e. 64trp2 (51 amino acids), 64trp3 (70 amino acids), 64trp1 (29 amino acids), 64trp4 (63 amino acids), 64trp5 (133 amino acids without HIS.TAG) and 64trp6 (133 amino acids with HIS.TAG), respectively. Predicted possible cleavage signal peptide (amino acids 1 to 18) is underlined in green.

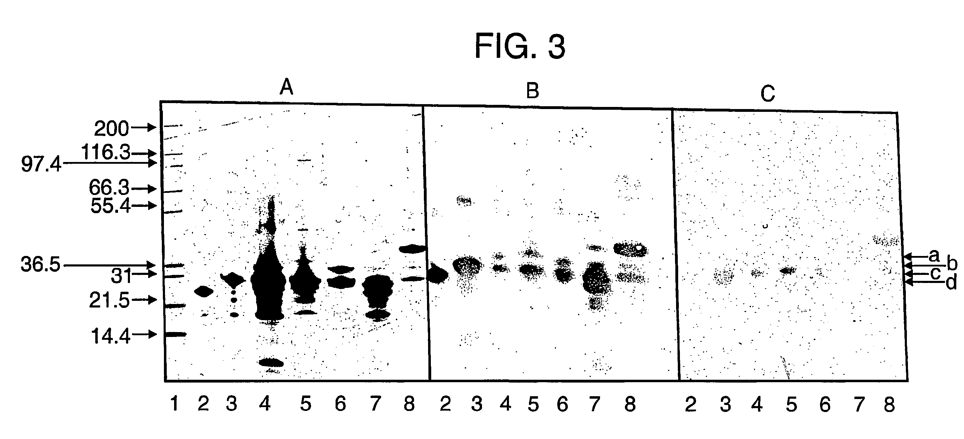

Figure 3: SDS-PAGE: (A) Coomassie Blue stained 4-12% gradient NuPAGE Bis-Tris gel, (B) and (C) Western Blots using GST monoclonal antibody (1:500 dilution) and HIS-TAG monoclonal antibody alkaline phosphatase conjugate (1:2000 dilution), respectively, of IPTG-induced *E. coli* cells expressing recombinant truncated versions (trp) of tick structural protein as well as vector-GST protein, 64P (i.e. trp1, trp2, trp3 and trp4). Lanes: 1=molecular weight markers, 2=26 kD vector-GST protein, 3=30 kD trp1 protein, 4=33 kD trp2 protein and 5=36 kD trp3 protein, 6=35 kD trp4 protein, 7=41 kD trp5 protein (none HIS-TAGGED) and 8=42 kD trp6 protein (HIS-TAGGED). Samples were solubilized at 100°C in SDS prior to loading the gels. Arrows: a=42 kD trp6 protein, b=35 kD trp4 protein, c=33 kD trp2 protein, d=30 kD trp1 protein and e=26 kD vector GST-protein.

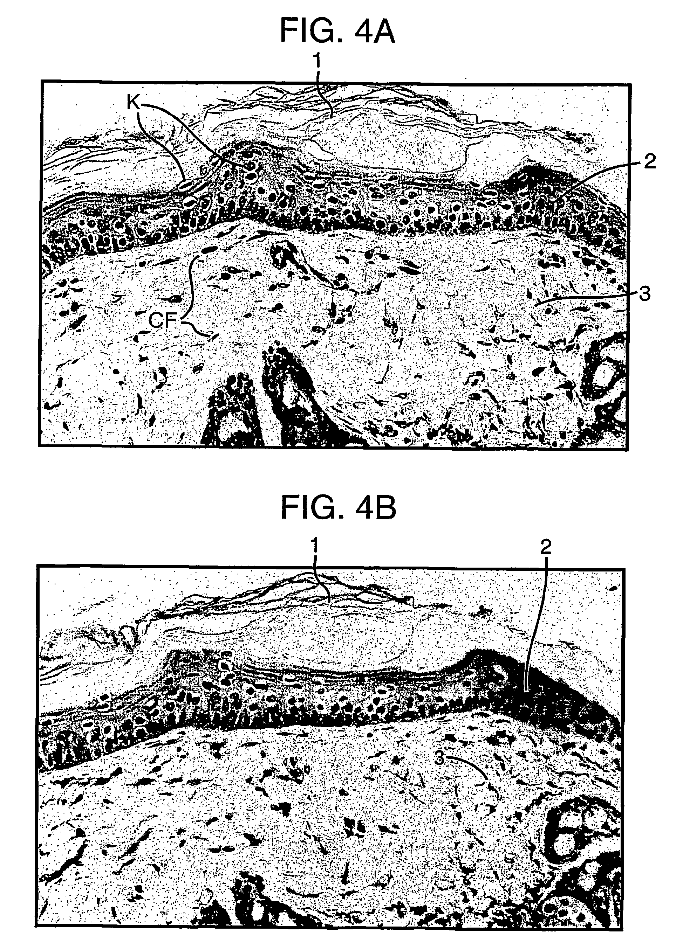

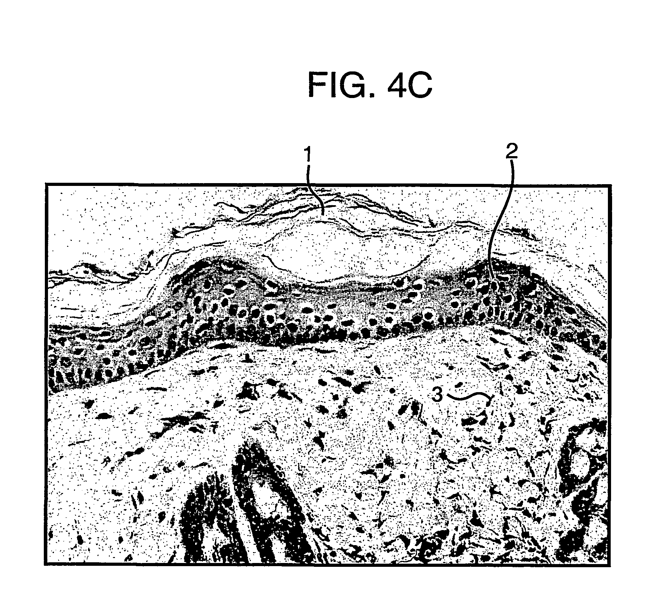

Figure 4: Immunoperoxidase studies using anti-64trp antisera on thin Hamster skin sections. A and C=thin Hamster skin sections incubated with anti-64trp antisera: 64trp3 and 64trp2, respectively, as primary antibodies; B=thin Hamster skin section incubated with PBS (physiologic saline solution) as primary antibody, i.e. control sample. 1=cornified layer of epidermis (stratum corneum), 2=epidermis and 3=dermis; K=keratinocytes and CF=collagen fibers, gave positive reaction (yellow/brown colour) with anti-64trp3 antiserum. Magnification=20X.

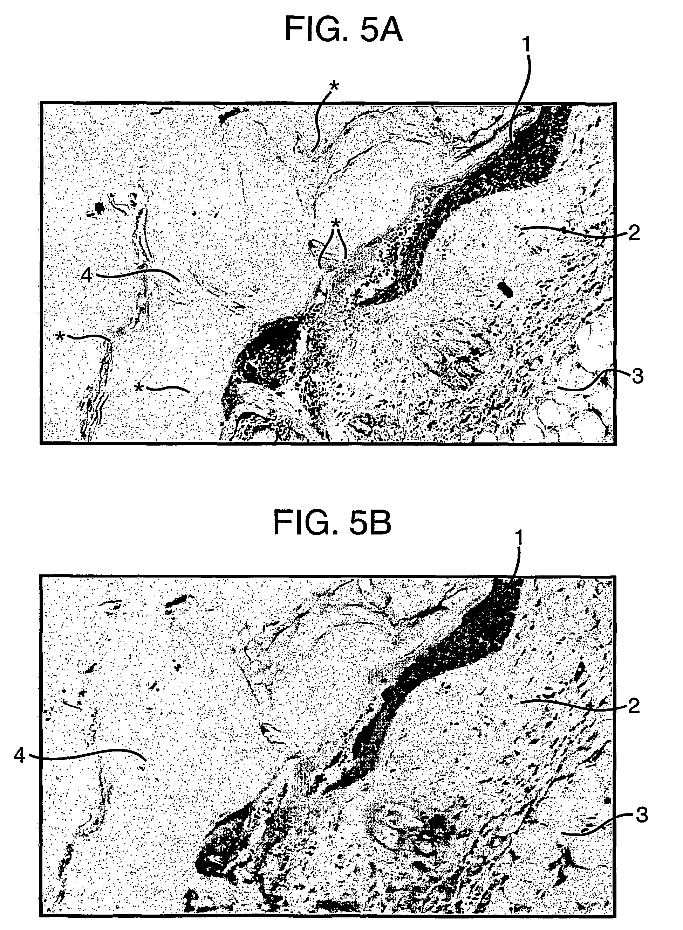

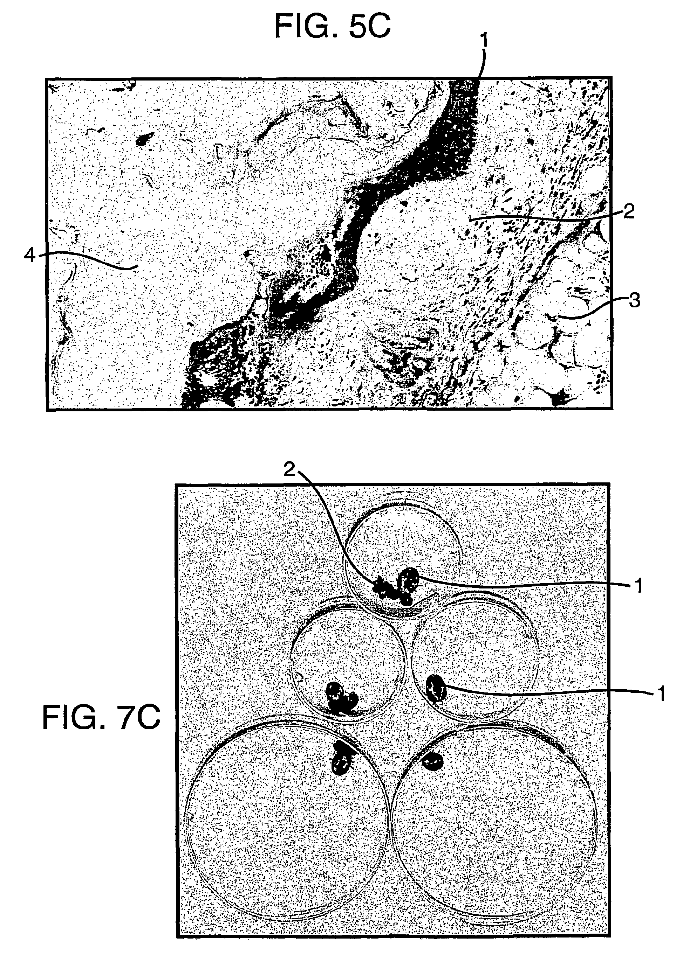

Figure 5: Immunoperoxidase studies using anti-64trp antisera on thin Hamster skin sections post feeding with *Rhipicephalus appendiculatus.* A and C = thin Hamster skin sections incubated with anti-64TRP anti-sera: 64trp3 and 64trp2, respectively, as primary antibodies; and B=hamster skin section incubated with PBS (physiological saline solution) in place of primary antibody, i.e. control sample. Arrows: 1=epidermis; 2=dermis; 3=subcutis; 4=tick cement cone; and *=sections of the cement cone and Hamster skin that gave positive reactions when incubated with anti-64trp3 antiserum. Magnification=10X.

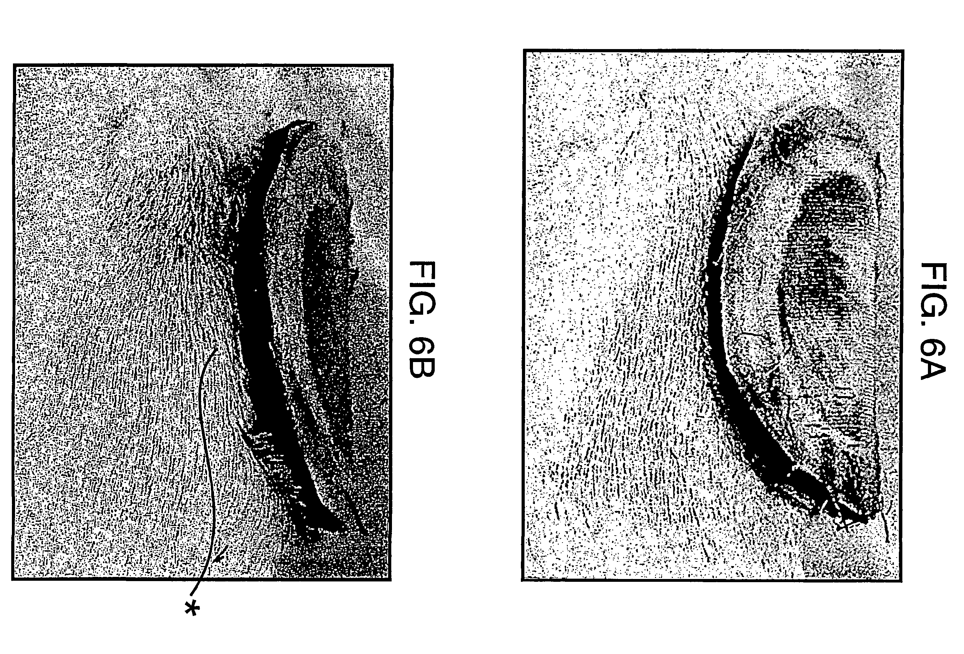

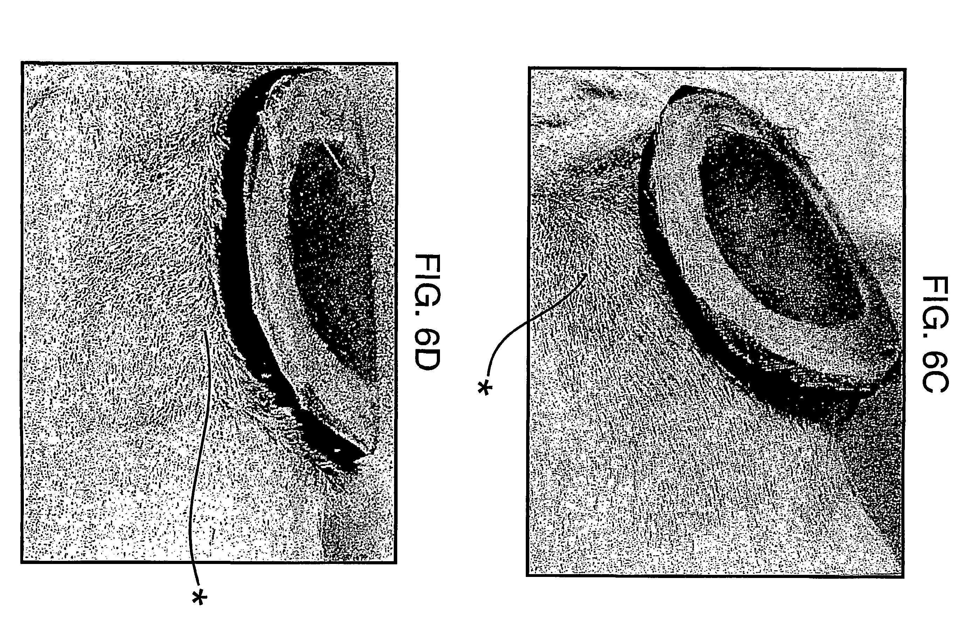

Figure 6: Effects of feeding *Rhipicephalus appendiculatus* nymphs on guinea pigs immunised with truncated versions of the 64P protein (64TRPs). A=cell with *Rhipicephalus appendiculatus* nymphs feeding on GST-immunised, control guinea pig; B, C and D=cells with *Rhipicephalus appendiculatus* nymphs feeding on guinea pigs immunised with 64trp proteins. *=arrow indicating sites of inflammation (i.e.erythema, oedema, lymphadenopathy and warm to touch) on skin of immunised guinea pigs B, C and D on which *Rhipicephalus appendiculatus* nymphs were feeding.

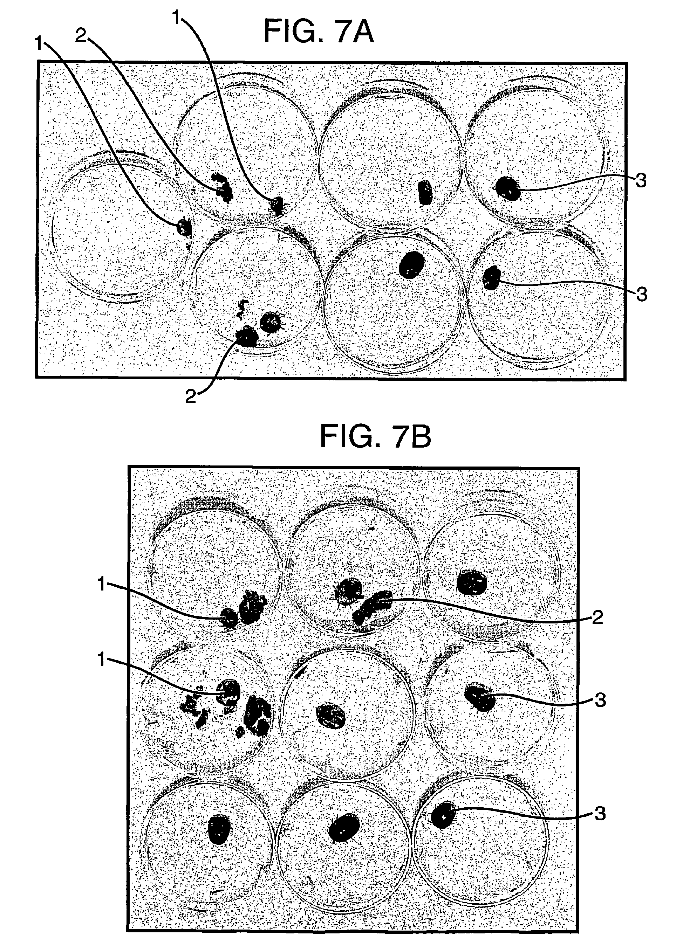

Figure 7: Effects on *Rhipicephalus appendiculatus* female adult ticks, post-feeding on guinea pigs immunised with 64trp proteins. A and B=*Rhipicephalus appendiculatus* female ticks, post-feeding on guinea pigs immunised with 64trp2 and 64trp3 proteins, respectively; and C=*Rhipicephalus appendiculatus* female ticks, post-feeding on GST-immunised, control guinea pigs. 1=live female ticks, 2=eggs, and 3=dead female ticks.

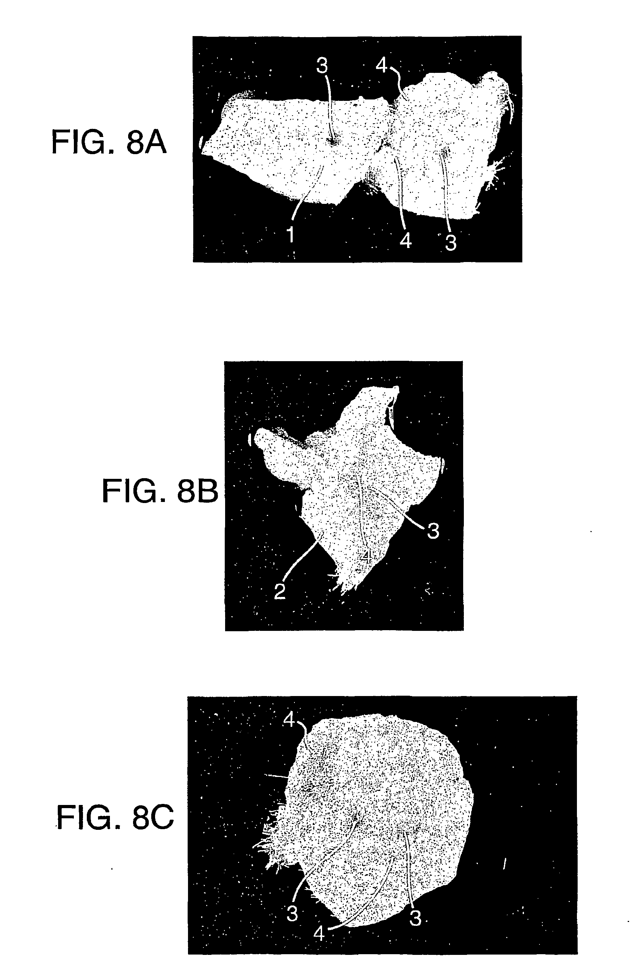

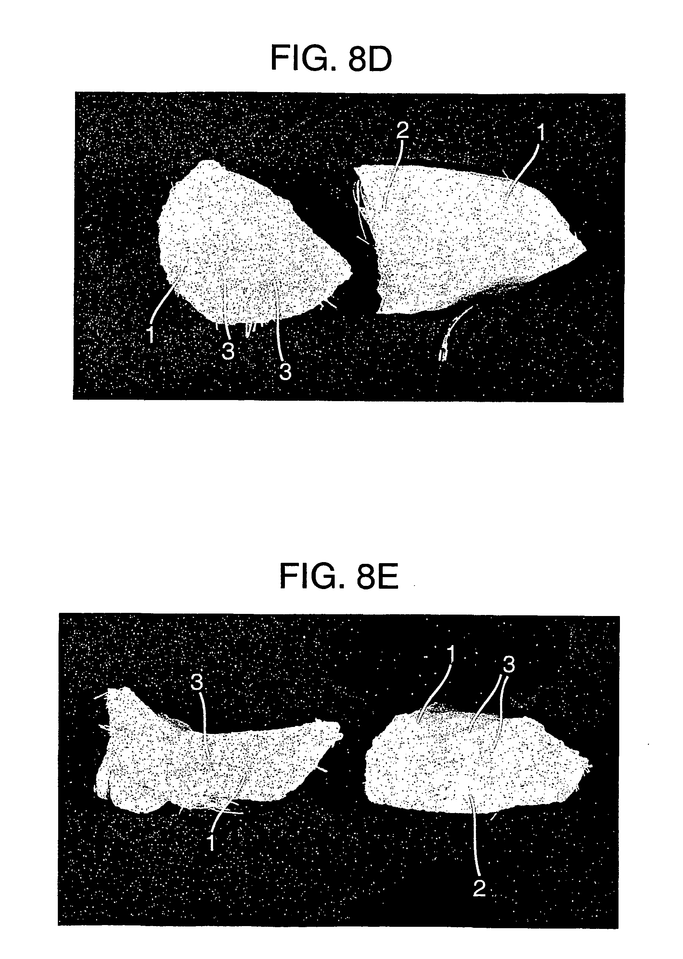

Figure 8: Necropsy studies of skin biopsies from guinea pigs immunised with 64trp proteins, post-feeding with *Rhipicephalus appendiculatus* ticks. A, B and C=skin biopsies from guinea pigs immunised with 64trp1, 64trp6 and 64trp2 proteins respectively, and, D and E=skin biopsies from control, GST-immunised guinea pigs, post-feeding with *Rhipicephalus appendiculatus* ticks; 1=epidermis, 2=dermis/subcutis, 3=previous tick attachment sites, 4=necrotic lesions.

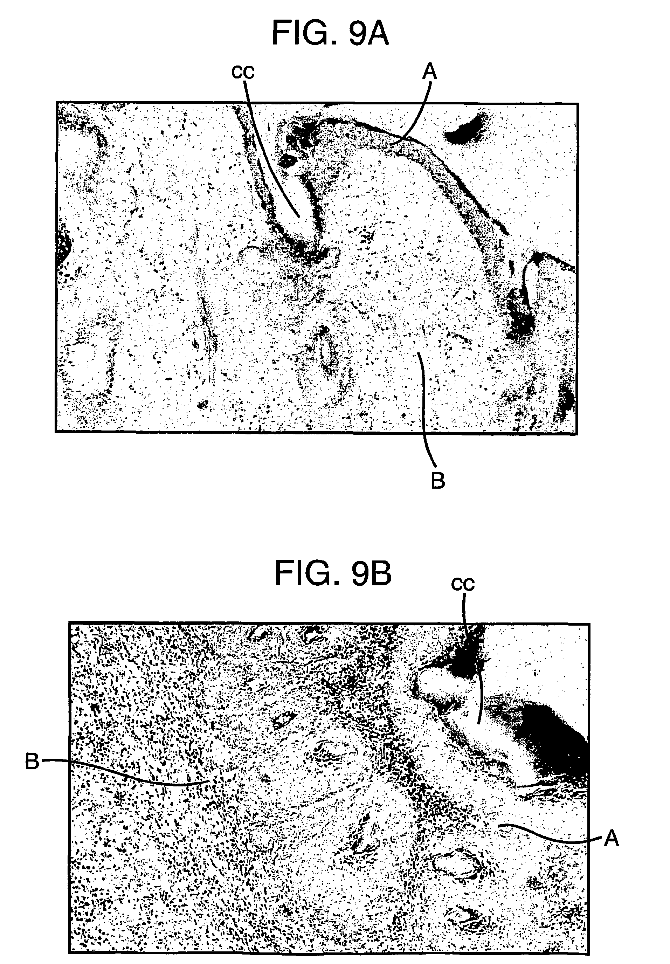

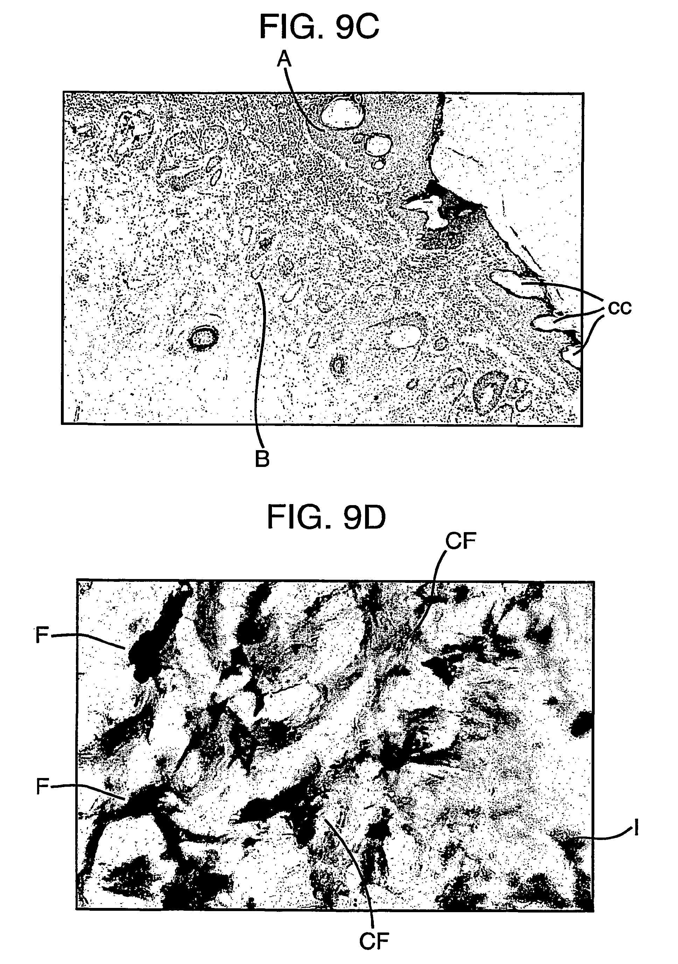

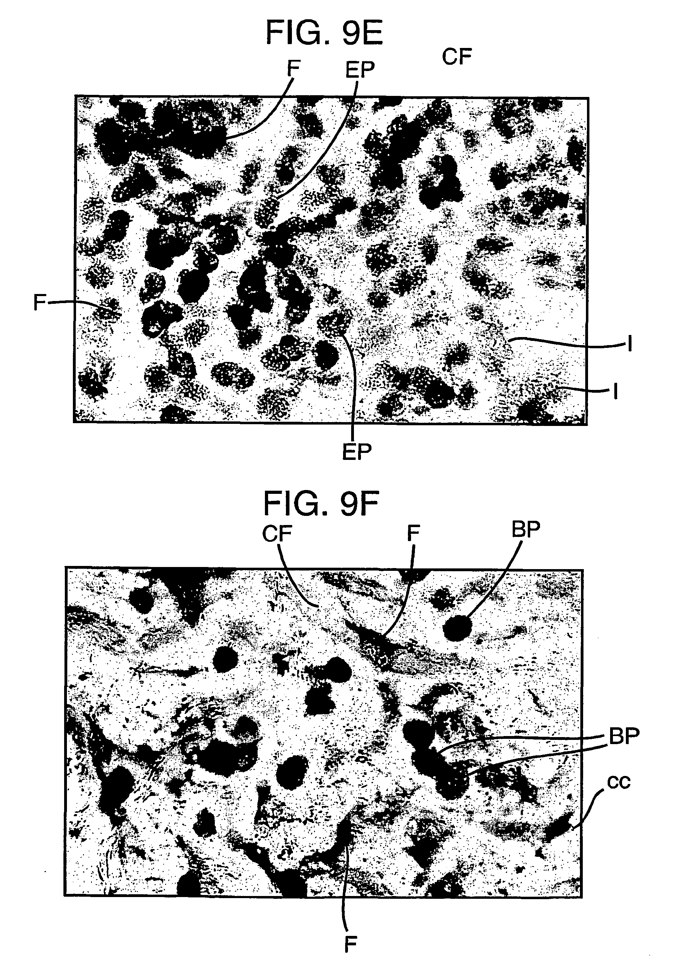

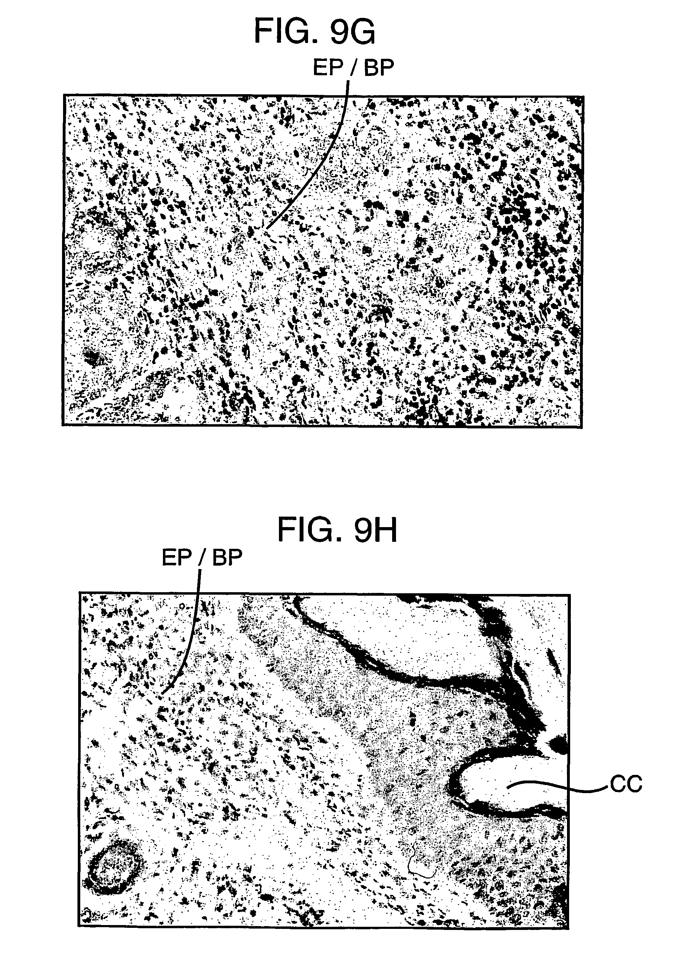

Figure 9: Histological studies of skin sections from guinea pigs immunised with 64trp proteins, post feeding with *Rhipicephalus appendiculatus* ticks, stained with Haematoxylin and Eosin, and Wright's stains. 1=histological section from skin of GST-immunised, control guinea pig and 2,3,4,5,6,7 and 8=histological sections from skin of Guinea pigs immunised with 64trp proteins, post-feeding with *Rhipicephalus appendiculatus* adult ticks, stained with: haematoxylin and eosin stains=sections 1,2,3,7 and 8, and Wright's stain=sections 4,5 and 6; sections 1,2 and 3-magnification=10X; sections 7 and 8-magnification=20X; sections 4,5, and 6-magnification=100X. Arrows: A=epidermis, B=dermis, cc=areas where *Rhipicephalus appendiculatus* tick cement cones were previously attached, CF=collagen fibers, D=dendrocytes, F=fibroblasts, EP=eosinophil polymorphs, BP=basophil polymorphs.

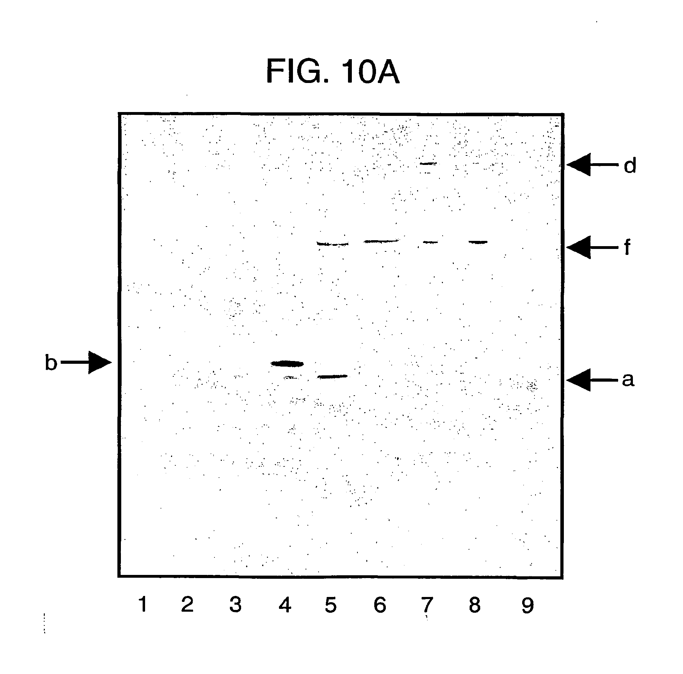

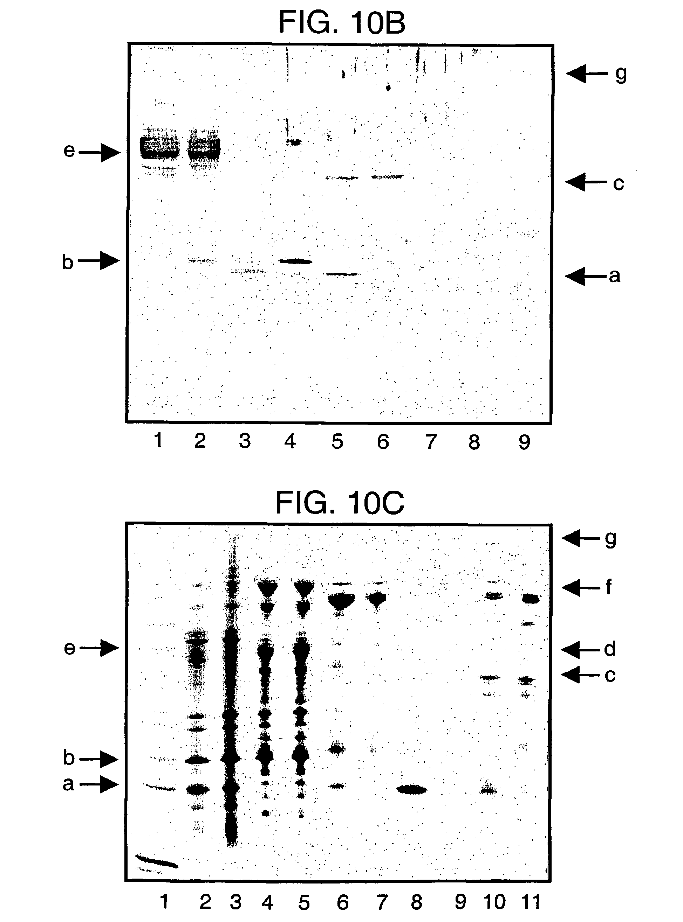

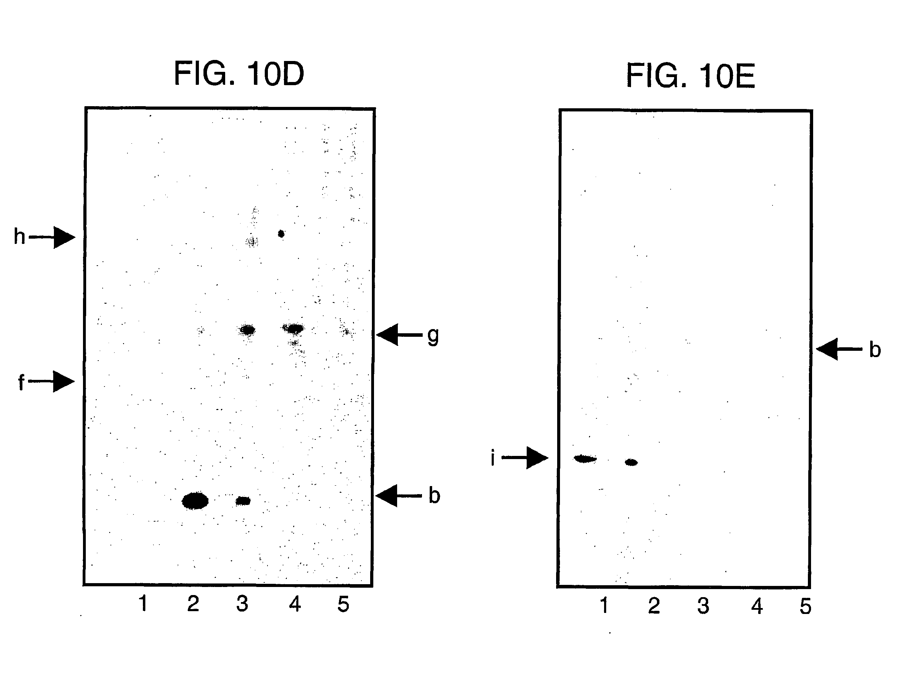

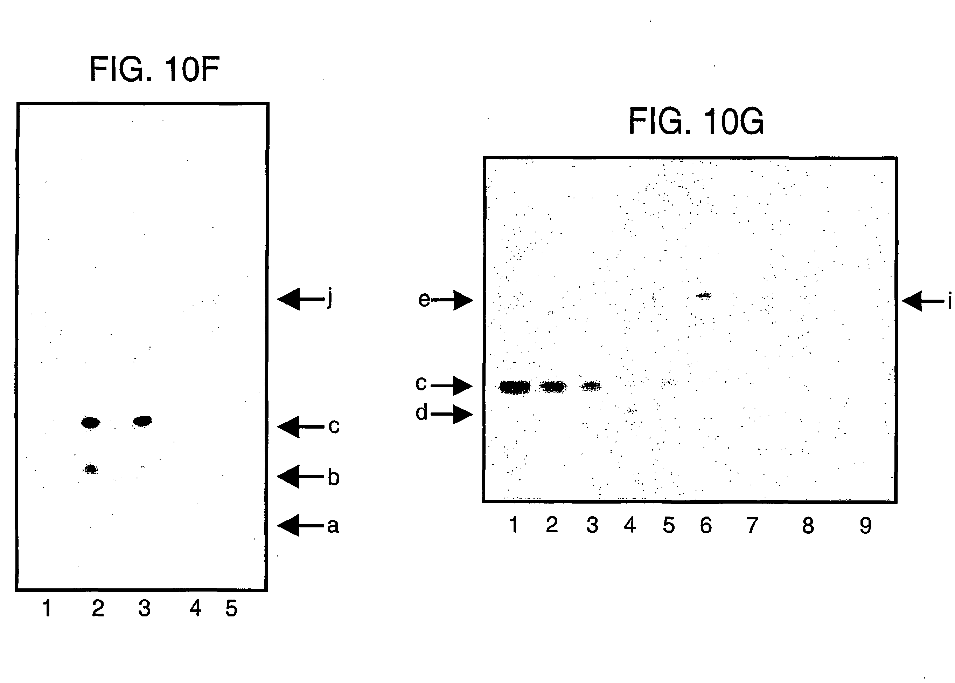

Figure 10: Cross-reactivity between soluble fractions of *R. appendiculatus* tick antigens using sera from guinea pig immunised with 64trp recombinant proteins.
   A Immunoblot of R. appendiculatus tick antigens probed with anti-64trp2 serum. Lanes A/1 and A/2 cement cone; Lanes A/3 and A/4 salivary glands; Lanes A/5 and A/6 haemolymph; Lane A/7 nymphs; Lane A/8 larvae; Lane A/9 molecular weight markers*.
   B Immunoblot of R. appendiculatus tick antigens probed with anti-64trp6 serum. Lanes B/1 and B/2 cement cone; Lanes B/3 and B/4 salivary glands; lanes B/5 and B/6 haemolymph; Lane B/7 nymphs; Lane B/8 larvae; Lane B/9 molecular weight markers*.
   C Coomassie Blue stained 4-12 % gradient gel of R. appendiculatus tick antigens (female & male). Lane C/1 molecular weight markers**; Lanes C/2 and C/3 cement cones; Lanes C/4 and C/5 salivary gland extracts; Lanes C/6 and C/7 haemolymph; Lanes C/8 and C/9 midgut; Lane C/10 nymphs; Lane C/11 larvae.
   D Immunoblot of *R. appendiculatus* tick antigens probed with anti-64trp6 antiserum. Lane All molecular weight markers*; Lanes A/2 and A/3 salivary glands unfed tick; Lanes A4, A/5 midgut.
   E Immunoblot of *R. appendiculatus* tick antigens probed with anti-64trp2 antiserum. Lanes B/1 and B/2 salivary glands unfed tick; Lane B/3 and B/4 midgut; Lane 5 molecular weight markers*
   F Immunoblot of *R. appendiculatus* tick antigens using anti-64trp5 antiserum. Lane C/1 molecular weight markers*; Lanes C/2 and C/3 salivary glands unfed tick; Lanes C/4 and C/5 midgut.
   G Immunoblot of *R. appendiculatus* tick antigens using anti-64trp5 antiserum. Lanes D/1 and D/2 cement cone; Lanes D/3 and D/4 salivary glands of partially fed (day 2) ticks; Lanes D/5 and D/6 haemolymph; Lanes D/7 nymphs; Lanes D/8 larvae; Lane D/9 molecular weight markers*.

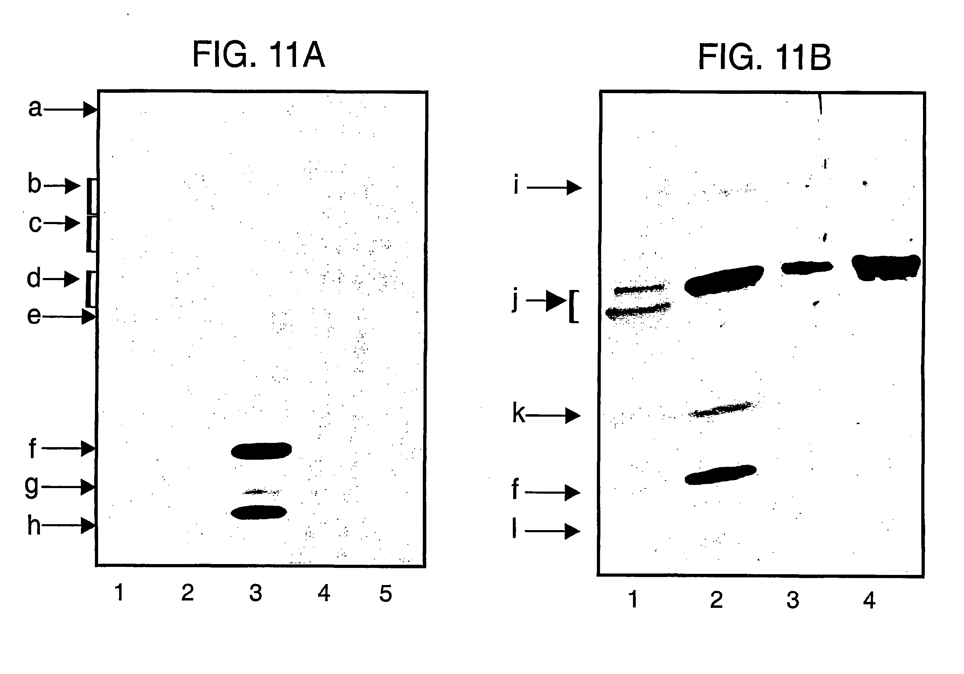

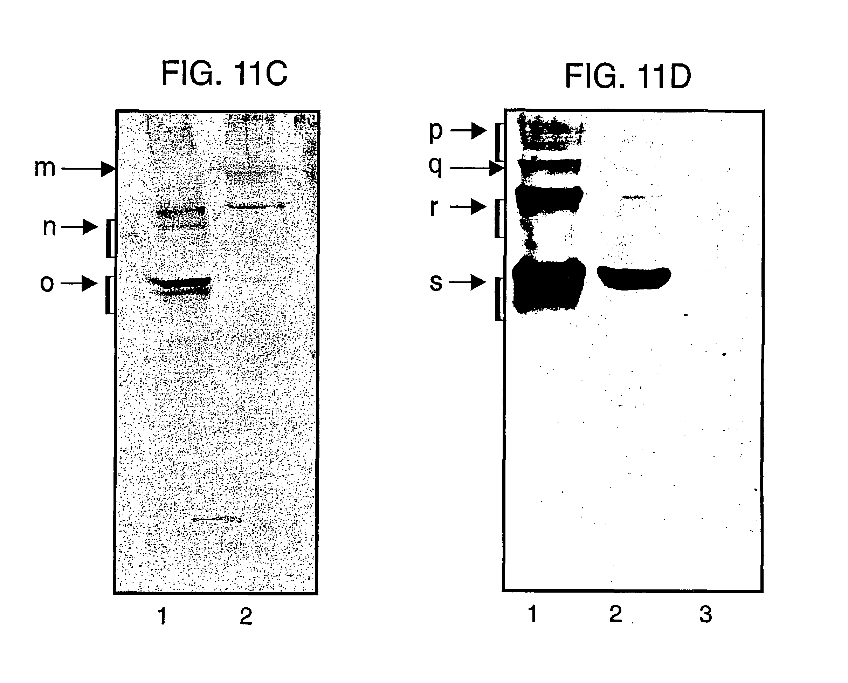

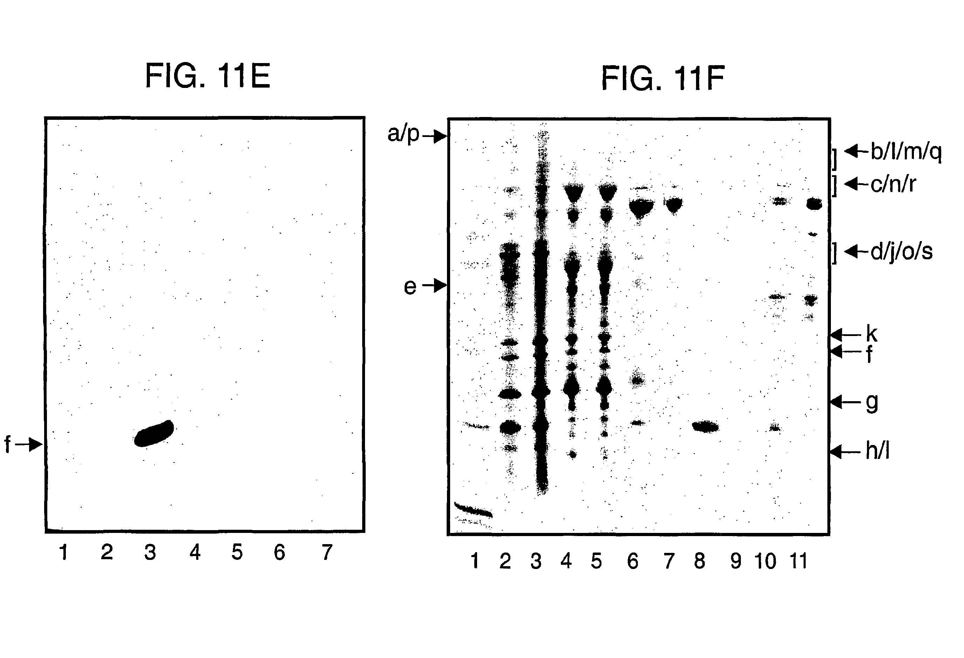

Figure 11: Cross-reactivity between insoluble fractions of *R. appendiculatus* tick antigens using sera from guinea pig immunised with 64trp recombinant proteins.
   A Immunoblot of *R. appendiculatus* tick antigens from adult female tissue extracts probed with anti-64trp3 serum. Lane A/1 markers***; Lane A/2 cement cone (from females partially fed on guinea pigs), Lane A/3 unfed tick salivary gland, Lane A/4 unfed tick haemolymph, Lane A/5 unfed tick midgut.
   B Immunoblot of *R. appendiculatus* tick antigens from adult female tissue extracts probed with anti-64trp2 serum. Lanes B/1, B/2, B/3 and B/4 as in Lanes A/2-5.
   C Immunoblot of *R. appendiculatus* tick antigens probed with anti-64trp6 serum. Lane C/1 whole nymphs, Lane C/2 whole larvae.
   D Immunoblot of *R. appendiculatus* tick antigens probed with anti-64trp2 serum. Lane D/1 whole nymphs, Lane D/2 whole larvae, Lane D/3 markers***.
   E Immunoblot of *R. appendiculatus* tick antigens probed with control anti-GST serum. Lane E/1, markers***, Lane E/2 cement cone (as Lane A/2), Lane E/3 salivary gland (as lane A/3), Lane E/4 haemolymph (as Lane A/4), Lane E/5 midgut (as Lane A/5), Lane E/6 nymph (as Lanes C/2, D/2), Lane E/7 larvae (as Lanes C/1, C/2).
Figure 11 A to E markers*** SeeBlue^{™} Plus2 protein molecular weight markers: 188 kD=Myosin, 98 kD=Phosphorylase B, 62kD=Bovine serum albumin, 49 kD=Glutamic Dehydrogenase, 38 kD=Alcohol Dehydrogenase, 28 kD=Carbonic Anhydrase, 17 kD=Myoglobin Red and 14 kD=Lysozyme.
Figure 11F Coomassie Blue stained 4-12 % gradient gel of *R. appendiculatus* tick antigens. Lanes F/1 markers**, Lane F/2 cement cone of partially fed males, Lane F/3 cement cone of partially fed females, Lane F/4 unfed male salivary glands, Lane F/5 unfed female salivary glands, Lane F/6 male haemolymph, Lane F/7 female haemolymph, Lane F/8 male midgut, Lane F/9 female midgut, Lane F/10 whole nymphs, Lane F/11 whole larvae. The 26 kD band f, a cross-reaction in the salivary glands, may represent *R. appendiculatus* GST equivalent to that reported for *Boophilus microplus* (He et al., (1999). Insect Biochem. Mol. Biol. 29: 737-743).
Figure 11 A to F. Molecular weights of labelled bands: **a**=200 kD, **b**=120-188 kD, **c**=80-98 kD, **d**=55-62 kD, **e**=49-55 kD, **f**=26 kD, **g**=17 kD, **h**=15 Kd, **i**=120 kD, **j**=60-62 kD, **k**=36 kD, **1**=14 kD, **m**=188 kD, **n**=98-120 kD, **o**=55-62 kD, **p**=200 kD, **q**=188 kD, **r**=150 kD & s=50-62 kD.
Figure 12: Cross-reactivity of *Amblyomma variegatum* and *Rhipicephalus sanguineus* tick antigens with antisera from guinea pigs immunised with recombinant R. appendiculatus 64trp proteins.
   A Immunoblot of *A. variegatum* tick antigens probed with anti-64trp5 serum. Lane A/1haemolymph; LaneA/2midgut; Lane A/3 salivary gland; Lane A/4 molecular weight markers*.
   B Immunoblot of *A. variegatum* tick antigens probed with anti-64trp6 serum. Lane A/1 molecular weight markers* LaneA/2 salivary gland; Lane A/3 midgut; Lane A/4haemolymph.
   C Immunoblot of *R. sanguineus* tick antigens probed with anti-64trp6 serum. Lane A/1 molecular weight markers* LaneA/2 salivary gland; Lane A/3 midgut; Lane A/4haemolymph
   For Figures 10-12, * represents MultiMarkTM protein molecular weight markers (NOVEX) : 98 kDa =Phosphorylase B, 52 kDa =Glutamic Dehydrogenase, 31 kDa =Carbonic Anhydrase, 19/17 kDa =Myoglobin Red/Blue, 11 kDa =Lysozyme, 6 kDa =Aprotinin, 3 kDa =Insulin
   **represents Mark 12TM protein molecular weight markers (NOVEX) :200 kDa=Myosin, 116.3 kDa=ß galactosidase, 97.4 kDa=Phosphorylase b, 66.3 kDa=Bovine serum albumin, 55.4 kDa=Glutamic dehydrogenase, 36.5 kDa=Lactate dehydrogenase, 31 kDa=Carbonic anhydrase, 21.5 kDa=Trypsin inhibitor, 14.4 kDa=Lysozyme, 6 kDa=Aprotinin, 3.5 kDa=Insulin B chain and 2.5 kDa=Insulin A chain.

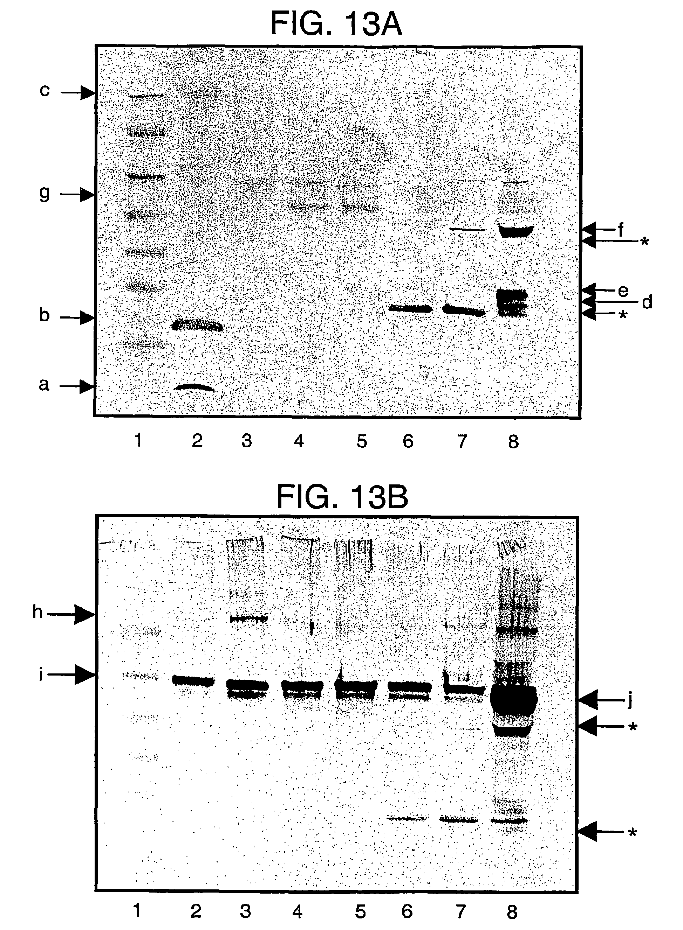

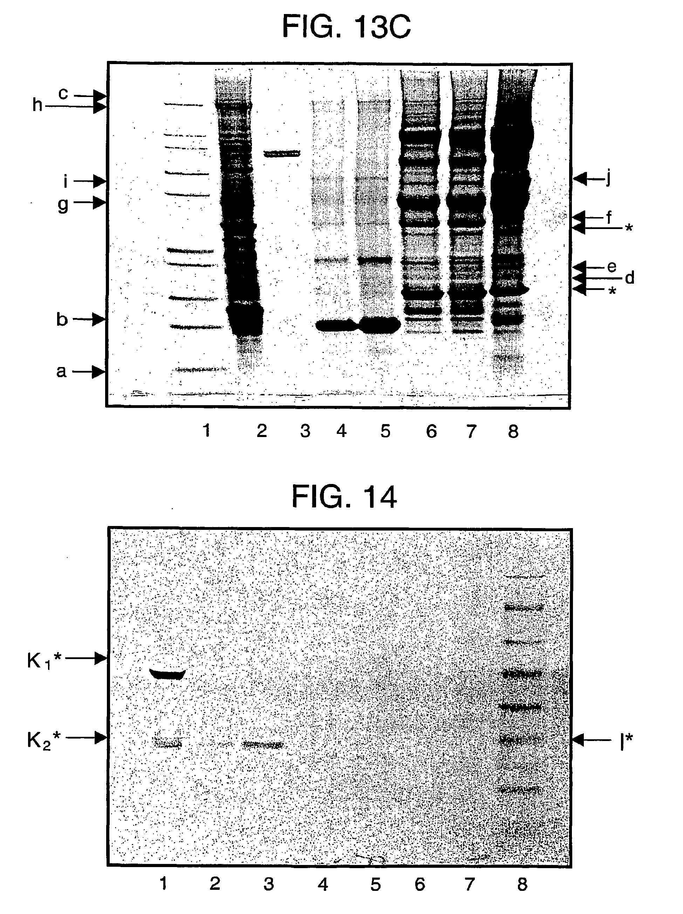

Figure 13: Cross-reactivity between *Rhipicephalus sanguineus* tick antigens using antisera from guinea pigs immunised with recombinant *R*. *appendiculatus* 64trp proteins.
   **A** and **B** Immunoblots of *R*. *sanguineus* tick antigens using anti-64trp3 and anti-64trp2 antisera, respectively. C Coomassie Blue stained 4-12% Bis-Tris gradient gel (NuPAGE-NOVEX).
   Lanes **A/1** and **B/1**: SeeBlue^{™} Plus2 protein molecular weight markers (NOVEX) 188 kD=Myosin; 98 kDa= Phosphorylase B; 62 kD=BSA; 49 kD=Glutamic Dehydrogenase; 38 kD=Alcohol Dehydrogenase; 28 kD=Carbonic Anhydrase; 17 kD=Myoglobin Red; 14 kD=Lysozyme; 6 kD=Aprotinin.
   Lane **C/1**: Mark12^{™} protein molecular weight markers (NOVEX) 200 kD=Myosin; 116.3 kD=B-galactosidase; 97.4 kD=Phosphorylase b; 66.3 kD=Bovine serum albumin; 55.4 kD=Glutamic dehydrogenase; 36.5 kD=Lactate dehydrogenase; 31 kD=Carbonic anhydrase; 21.5 kD=Trypsin inhibitor; 14.4 kD=Lysozyme; 6 kD=Aprotinin.
   Lanes:**A/2**, **A/3**, **A/4**, **A/5**, **A/6**, **A/7** and **A/8**: *R. sanguineus* whole nymph extract, haemolymph, male midgut extract, female midgut extract, male salivary gland extract, female salivary gland extract and, combined male and female cement cone extract (from ticks partially fed on guinea pigs), respectively, of which immunopositive bands were observed as **a**=6 kD, **b**=17 kD, **c**=188 kD, **d**=26 kD, **e**=28 kD, **f**=60 kD and **g**=80 kD, using anti-64trp3 antiserum.
   Lanes:**B/2**, **B/3**, **B/4**, **B/5**, **B/6**, **B/7** and **B/8**: as in **A** of which immunopositive bands were observed as **h**=120 kD, **i**=62 kD and **j**=60 kD, using anti-64trp2 antiserum.
   Lanes:**C/2**, **C/3**, **C/4**, **C/5**, **C/6**, **C/7** and **C/8**: as in **A** of which protein bands **a** to **j** correspond to the immunopositive bands from immunoblots **A** and **B**. Arrow *=immunopositive bands due to background binding with anti-GST antiserum - see Figure 14.
Figure 14: Immunoblot of *R. sanguineus* tick antigens using anti-GST antiserum.
   Lane **8**: Mark12^{™} protein molecular weight markers (NOVEX) 200 kD=Myosin; 116.3 kD=B-galactosidase; 97.4 kD=Phosphorylase b; 66.3 kD=Bovine serum albumin; 55.4 kD=Glutamic dehydrogenase; 36.5 kD=Lactate dehydrogenase; 31 kD=Carbonic anhydrase; 21.5 kD=Trypsin inhibitor; 14.4 kD=Lysozyme; 6 kD=Aprotinin. Lanes **7**, **6**, **5**, **4, 3, 2**, and **1**: *R. sanguineus* whole nymph extract, haemolymph, male midgut extract, female midgut extract, male salivary gland extract, female salivary gland extract and, combined male and female cement cone extract (from ticks partially fed on guinea pigs), respectively. The immunopositive bands **I*** and **K1** and **K2*** refer to non-specific binding with the anti-GST antiserum with protein bands (25 kD, 48 kD and 26 kD) present in male and female salivary gland extracts, and cement cone extract, respectively.

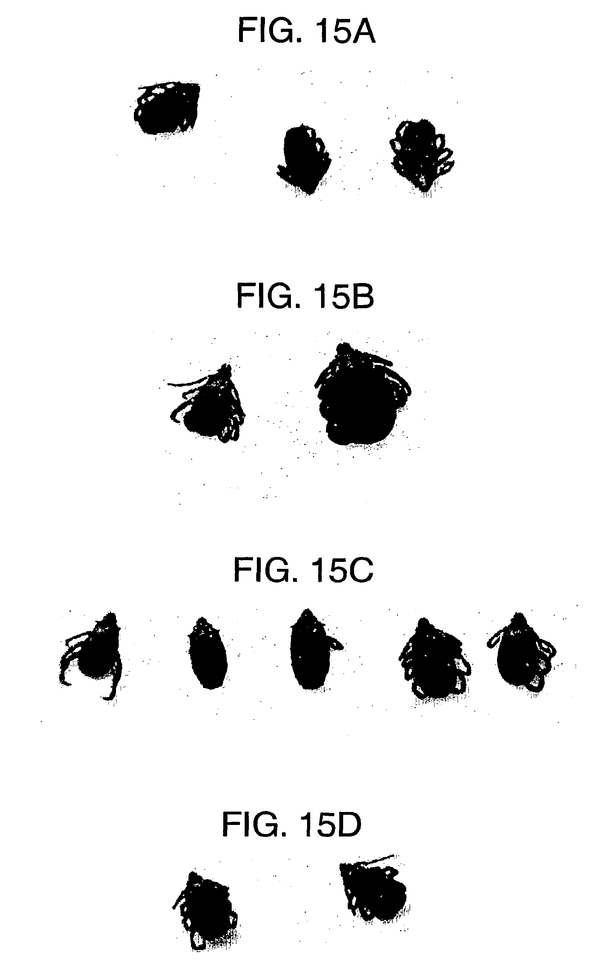

Figure 15: Effects on *Rhipicephalus sanguineus* male and female adult ticks during early-feeding on guinea pigs immunised with different cocktails of 64trp proteins.
   (**A**) dead female ticks from 64trp6/3 - immunised guinea pigs, (injected into separate sites);
   (**B**) dead female ticks from 64trp6/3 - immunised guinea pigs, (combined and injected into a single site);
   (**C**) dead female (n=3) and male (n=2) ticks from 64trp6/2 - immunised guinea pigs, ( injected into separate sites); (**D**) dead female ticks from 64trp6/2 - immunised guinea pigs, (combined and injected into a single site).

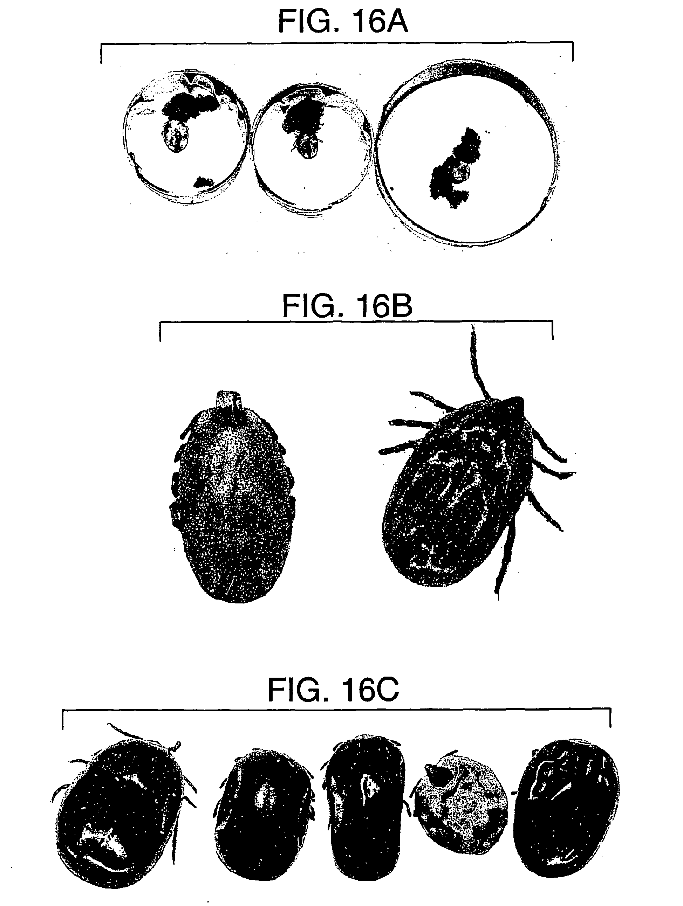

Figure 16: Vaccine effect of 64trp proteins on *Rhipicephalus sanguineus* adult ticks post-feeding on 64trp-immunised guinea pigs.
   (**A**): Surviving adult female *R. sanguineus* ticks, post-feeding on guinea pigs immunised with 64trp2/6 protein-cocktail as immunogen. Only 2/10 ticks survived; ticks did not lay eggs and did not fully engorge.
   (**B**): Dead adult female *R. sanguineus* ticks, post-feeding on guinea pigs immunised with 64trp2/6 and 64trp3/6 cocktails as immunogens. Ticks turned brown/black and developed a dry consistency 2-5 days post-detachment.
   (**C**): Normal adult *R. sanguineus* female ticks laying eggs, post-feeding on guinea pigs immunised with GST control protein.

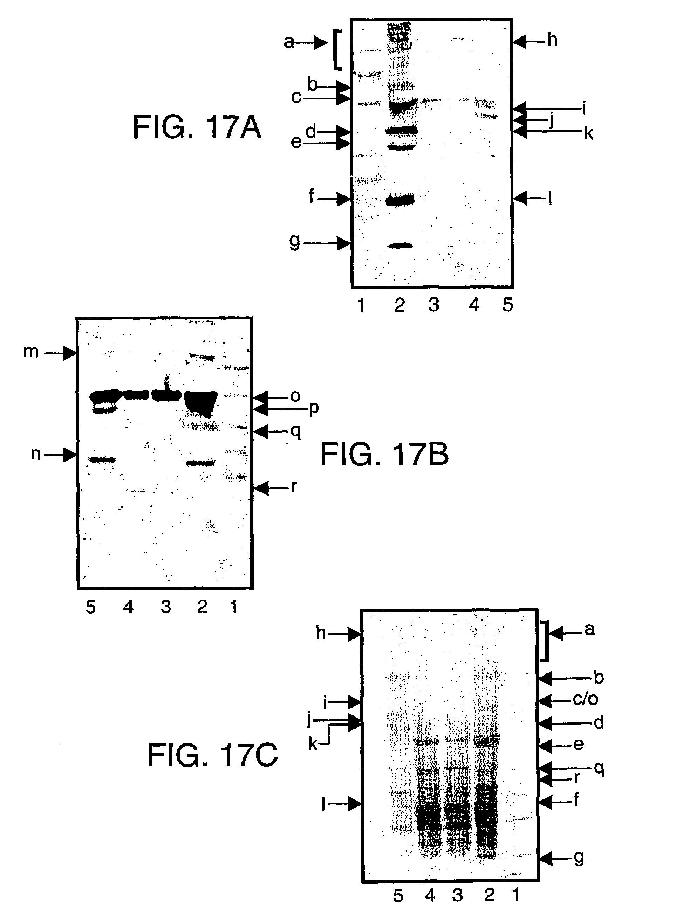

Figure 17: Cross-reactivity between *Boophilus microplus* tick antigens using antisera from guinea pigs immunised with recombinant *R. appendiculatus* 64trp2 and 64trp3 proteins **A** and **B** immunoblots of *Boophilus microplus* tick antigens using 64trp2 and 64trp3 antisera, respectively; **C** Coomassie Blue stained 4-12% Bis-Tris gradient gel (NuPAGE-NOVEX).
   Lanes: **A/1** and **B/1**: SeeBlue^{™} Plus 2 protein molecular weight markers (NOVEX) 188 kD=Myosin, 98 kD=Phosphorylase B, 62 kD=BSA, 49 kD=Glutamic Dehydrogenase, 38 kD=Alcohol Dehydrogenase, 28 kD=Carbonic Anhydrase, 17 kD=Myoglobin Red, 14 kD=Lysozyme, 6 kD=Aprotinin. Lane C/1: Mark12^{™} protein molecular weight markers (NOVEX): 200 kD=Myosin, 116.3 kD=B-galactosidase, 97.4 kD=Phosphorylase b, 66.3 kD=Bovine serum albumin, 55.4 kD=Glutamic dehydrogenase, 36.5 kD=Lactate dehydrogenase, 31 kD=Carbonic anhydrase, 21.5 kD=Trypsin inhibitor, 14.4 kD=Lysozyme and 6 kD=Aprotinin.
   Lanes **A/5**, **A/4**, **A/3** and **A/2**: *Boophilus microplus* cement cone extract, salivary gland extract, midgut extract and whole nymph (fed ticks) extract, respectively, of which immunopositive bands were observed as **a**=120-200 kD, **b**=80 kD, **c**=62 kD, **d**=49 kD, **e**=40 kD, **f**=17 kD, **g**=8 kD and **h**=200 kD, **I**=60 kD, **j**=55 kD, **k**=49 kD and **l**=1 kD using 64trp2 antiserum.
   Lanes **B/5**, **B/4**, **B/3** and **B/2**: *Boophilus microplus* cement cone extract, salivary gland extract, midgut extract and whole nymph extract (fed ticks), respectively, of which immunopositive bands were observed as **m**=120 kD, **n**=35 kD, **o**=62 kD, **p**=59 kD, **q**=49 kD and **r**=26 kD using 64trp3 antiserum.
   Lanes **C/5**, **C/4**, **C/3** and **C/2**:*Boophilus microplus* cement cone extract, salivary gland extract, midgut extract and whole nymph extract (fed ticks), respectively, of which protein bands **a** to **r** correspond to the immunopositive bands from immunoblots **A** and **B**.

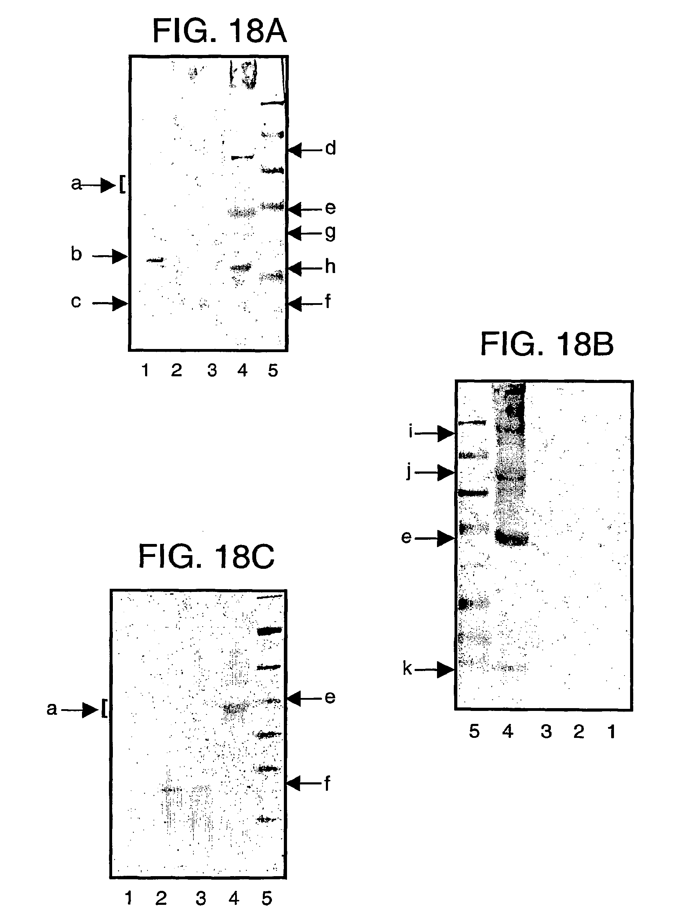

Figure 18: Cross-reactivity between *Boophilus microplus* tick antigens using antisera from guinea pigs immunised with recombinant R. appendiculatus 64trp5 and 64trp6 proteins.
   **A** and **B** immunoblots of *Boophilus microplus* tick antigens using anti-64trp5 and anti-64trp6 antisera, respectively; **C** immunoblot of *Boophilus microplus* tick antigens using antisera from guinea pigs immunised with recombinant GST protein.
   Lanes: **A/5**, **B/5**and **C/5**=SeeBlue^{™} Plus 2 protein molecular weight markers (NOVEX) : 188 kD=Myosin, 98 kD=Phosphorylase B, 62 kD=BSA, 49 kD=Glutamic Dehydrogenase, 38 kD=Alcohol Dehydrogenase, 28 kD=Carbonic Anhydrase, 17 kD=Myoglobin Red,14 kD=Lysozyme, 6 kD=Aprotinin.
   Lanes: **A/1**, **A/2**, **A/3** and **A/4**: *Boophilus microplus* cement cone extract, salivary gland extract, midgut extract and whole nymph (fed ticks) extract, respectively, of which immunopositive bands were observed as **a**=50-55 kD, **b**=32 kD, **c**=17 kD, **d**=70 kD, **e**=48 kD, **f**=26 kD, **g**=40 kD and **h**=30 kD, using anti-64trp5 antiserum.
   Lanes: **B/1**, **B/2**, **B/3** and **B/4**: *Boophilus microplus* cement cone extract, salivary gland extract, midgut extract and whole nymph (fed ticks) extract, respectively, of which immunopositive bands were observed as **e**=48 kD, **i**=180 kD, **j**=75 kD, **k**=12 kD, using anti-64trp6 antiserum.
   Lanes: **C/1**, **C/2**, **C/3** and **C/4**: *Boophilus microplus* cement cone extract, salivary gland extract, midgut extract and whole nymph (fed ticks) extract, respectively, of which immunopositive bands were observed as **a**= 50-55 kD, **e**=48.

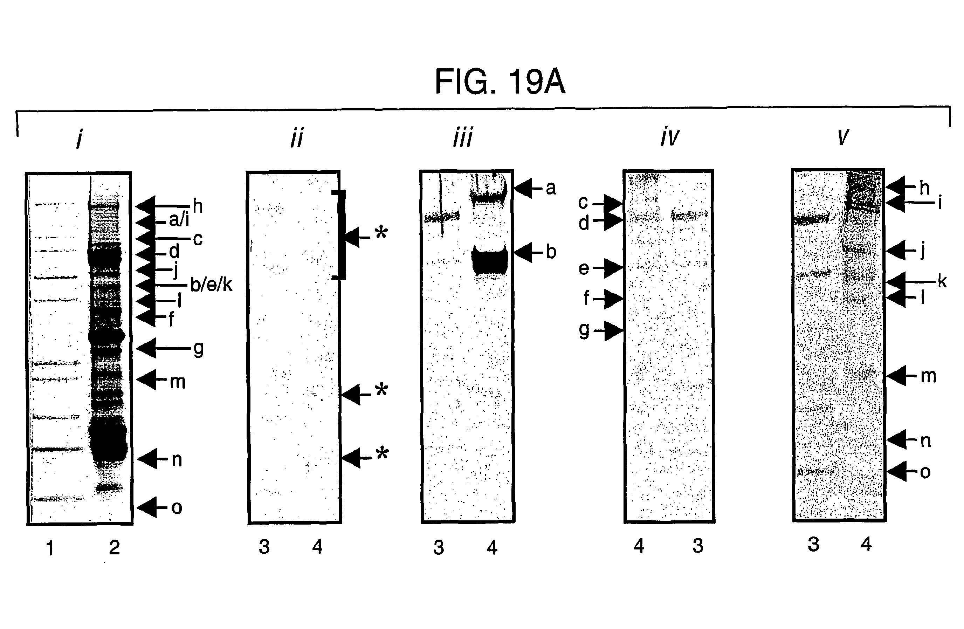

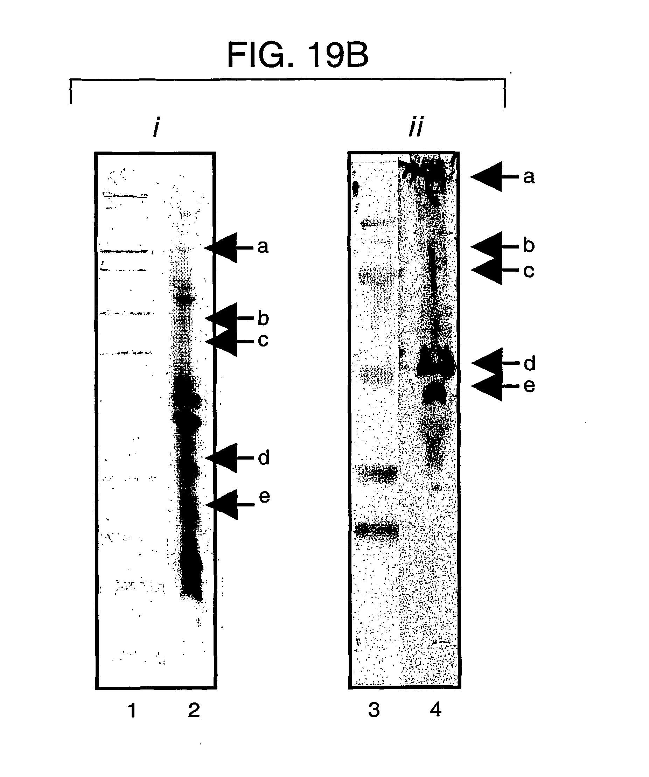

Figure 19: Cross-reactivity between *Ixodes ricinus* tick antigens using antisera of from guinea pigs immunised with recombinant *R. appendiculatus* 64trp proteins.
   **A (ii)**, **(iii)**, **(iv)** and **(v)** immunoblots of whole nymph extract of *Ixodes ricinus* unfed ticks using GST, 64trp2, 64trp5 and 64trp6 antisera, respectively; **A (i)** Coomassie Blue stained 4-12% Bis-Tris gradient gel (NuPAGE-Novex) of the same extract.
   **B(i)** and **(ii)** a Coomassie Blue stained 4-12% Bis-Tris gradient gel (NuPAGE-Novex) and an immunoblot of whole larval extract of *Ixodes ricinus* ticks using 64trp6 antiserum, respectively.
   Lanes: **A/(i)/1** and **B/(i)/1**: Mark12^{™}protein molecular weight markers (NOVEX): 200 kD=Myosin, 116.3 kD=B-galactosidase, 97.4 kD=Phosphorylase b, 66.3 kD=BSA, 55.4 Kd=Glutamic dehydrogenase, 36.5 kD=Lactate dehydrogenase, 31 kD=Carbonic anhydrase, 21.5 kD=Trypsin inhibitor, 14.4 kD=Lysozyme and 6 kD=Aprotinin. Lanes: **A/(ii)**, **(iii)**, **(iv)** and **(v)/3**: See Blue^{™} Plus 2 protein molecular weight markers (Novex): 98 kD=Phosphorylase B, 62 kD=BSA, 49 kD=Glutamine Dehydrogenase, 38 kD=Alcohol Dehydrogenase, 28 kD=Carbonic Anhydrase, 17 kD=Myoglobin Red, 14 kD=Lysozyme.Lane: **B/(ii)/3**: MultiMark^{™} protein molecular weight markers (Novex) : 98 kD=Phosphorylase B, 52 kD=Glutamic dehydrogenase, 31 kD=Carbonic anhydrase, 19 kD=Myoglobin Red, 17 kD=Myoglobin Blue and 11 kD=Aprotinin.
   Lanes:**A/ (iii)**, **(iv)** and **(v)/4**=*I*. *ricinus* whole nymph extract of which immuno-positive bands were observed as **a**=150 kD and **b**=62 kD, **c**=100 Kd, **d**=98 Kd, **e**=62 Kd,**f**=50 kD and **g**=42 kD, **h**=190 kD**, i**=150 kD, **j**=80, **k**=62 kD, **1**=55 kD, **m**=32 kD, **n**=17 kD and **o**=12 kD , respectively. Lane: **A/(ii)/4**= *I. ricinus* whole nymph extract of which very faint immunopositive bands observed as * were due to non-specific cross-reactivity of tick antigens with GST antiserum.
   Lane: **B/(ii)/4**= *I. ricinus* whole larva extract of which immunopositive bands were observed as **a**=116 kD, **b**=80 kD, **c**=62 kD, **d**=34 kD and **e**=28 kD.

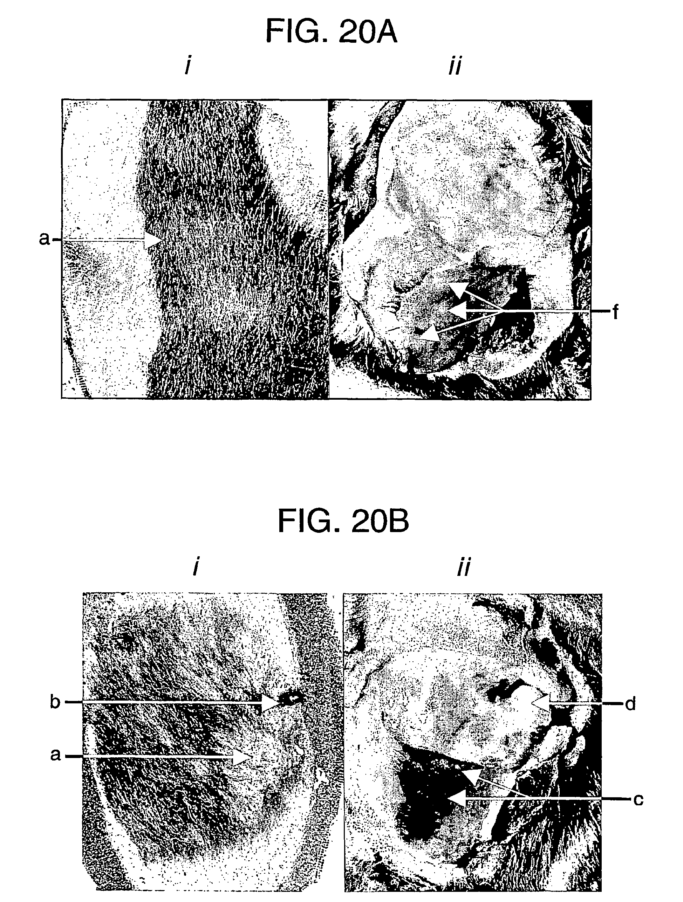

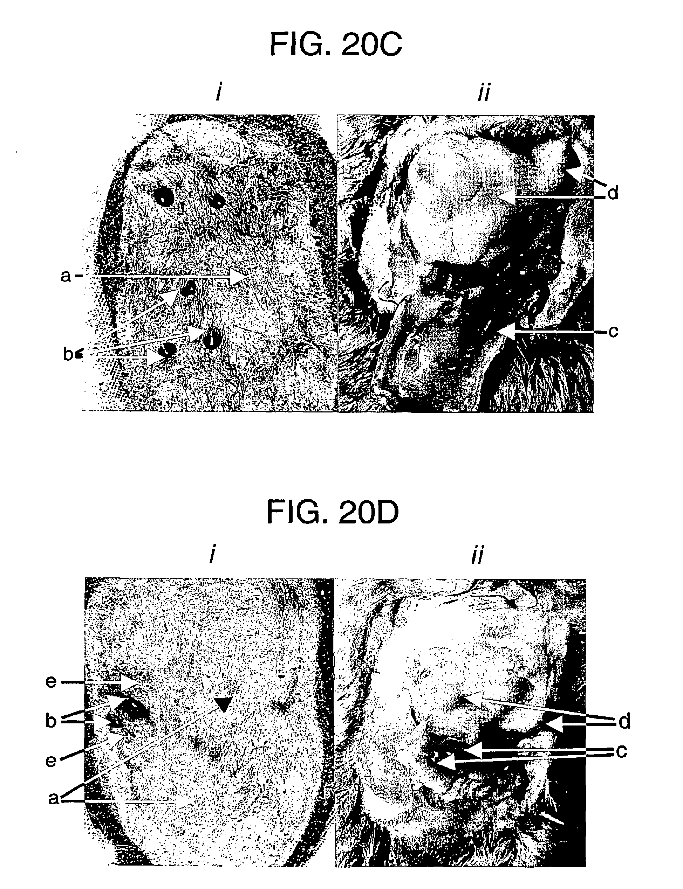

Figure 20: Effects of feeding nymphs of *Ixodes ricinus* ticks on hamsters immunised with different cocktails of 64trp proteins.

Studies on hamster skins during feeding [(i)-pre-dissection) and post-feeding ((ii)-post-dissection] of nymphs of *I*. *ricinus* ticks on hamsters immunised with GST control protein (**A**), 64trp6 protein (**B**), 64trp5 protein (**C**) and a cocktail of 64trp6/5 (**D**).

Hair growth post-tick feeding was normal in control hamster **A** and very slow for 64trp-immunised hamsters with areas of alopecia indicated by arrow **a.** This is due to the local inflammatory immune responses that occurred during the feeding of *I*. *ricinus* ticks on the 64trp-immunised hamsters, indicated by arrows: **c**, **d** and **e**; **c**=abandoned tick feeding sites showing various degrees of inflammation seen as erythema on the subcutis of the dissected skin; **d**=enlarged prescapular lymph nodes; **e**=serous exudation at abandoned tick feeding sites; **f**=abandoned tick feeding sites on the control hamster of which the subcutis and prescapular lymph nodes look normal. **b**=nymphs of *I*. *ricinus* ticks feeding; tick feeding on the control hamster was extended for 6 hours compared to those that fed on the 64trp-immunised hamsters.

### Examples

### Example 1: Expression of truncated cement protein (64TRP) in bacteria

As attempts to express the full-length clone of 64P in the baculovirus system were unsuccessful, several strategies were investigated for expressing truncated versions of 64P in *Escherichia coli* bacterial cells.

Several unsuccessful attempts were made to insert the entire coding region of the cement protein (minus the signal sequence, i.e. 144 amino acids in length) designated 64P, into pET23a(+) (Novagen) and pGEX-2T (Phamarcia) prokaryotic expression vectors, for expression of the protein in *E. coli* AD494 cells (Novagen). It was concluded that the structure of the construct may be toxic to the bacterial cell.

Therefore, a cloning strategy was adopted that involved serial cloning of truncated regions of the 64P clone, starting at the N-terminal region (Figure 2). Oligonucleotides were designed with appropriate restriction enzyme sites to permit PCR cloning of the different fragments of 64P from the cDNA. These constructs are as follows:
64trp1 = 29 amino acid C-terminal fragment
64trp2 = 51 amino acid N-terminal fragment
64trp3 = 70 amino acid N-terminal fragment
64trp4 = 63 amino acid C-terminal fragment
64trp5 = 133 amino acid fragment without 9XHIS.TAG
full-length 64P clone minus 3 amino acids at end of the sequence
64trp6 = 133 amino acid with the 9XHIS.TAG

The plasmids were transformed into *E. coli* XL1-BLUE cells (Stratagene) according to the manufacturer's instructions. The GST-fusion/histidine-tagged expressed 64TRP proteins were purified by the GST-purification method (Pharmacia) according to the manufacturer's instructions. The 9XHIS.TAG was included for ease of purification of 64TRP proteins that might be expressed as inclusion bodies (because the GST purification method applies to soluble proteins only).

Unlike the other GST/9XHIS.TAGGED truncated 64P proteins (i.e. 64trp1, 64trp2, 64trp3 and 64trp4), 64trp6 protein (133 amino acids with 9XHIS.TAG) resulted in the formation of inclusion bodies instead of soluble proteins. Therefore 64trp6 protein was purified under denaturing conditions using TALON metal affinity beads (Clontech) according to the manufacturer's recommendations.

The 64TRP proteins were analysed by NuPAGE Tris.Bis 4-12% gradient Coomassie Blue stained gels (Novex), according to the manufacturer's recommendations (Figure 3A). Figures 3B and 3C are Western Blots of similar gels from Figure 3A, using GST monoclonal antibody (GST mAb-Pharmacia) at a 1:500 dilution and 6XHIS.TAG monoclonal antibody (6XHIS.TAG mAb/APC-Clontech) at a dilution of 1:2,000, according to the manufacturers' instructions.

The expected 64TRP protein bands were observed on the Coomassie blue-stained gel (Figure 3A) with the following molecular weights: 64trp1 = 30 kDa; 64trp2 = 33 kDa; 64trp3 = 36 kDa; 64trp4 = 35 kDa; 64trp5 = 41 kDa; 64trp6 = 42 kDa.

Expression was confirmed on the Western Blots (Figures 3B and 3C). The 26 kDa GST protein band was used as a control marker.

As expected, the 64trp5 and GST protein bands each lacking the 9XHIS.TAG gave no reactions with the 6XHIS.TAG mAb (Figure 3C, lanes 7 and 2, respectively).

64TRP constructs that contained either a fragment or the complete N-terminal sequence of the 64P clone (i.e. 64trp2, 64trp3 and 64trp5), when expressed in E. coli XL1-BLUE cells, resulted in degradation products (Figures 3A, lanes 4, 5 and 7). This was confirmed by amino-terminal sequence analysis (Matsudaira, (1987) Journal of Biological Chemistry 262: 10035-10038) of the degraded protein bands. To try and resolve the problem of product degradation, the constructs will be transformed into *E. coli* BL21 strain, which carries a deletion in the ompT gene encoding a protease.

As expected, there was less degradation of 64trp6 protein compared to 64trp5 protein because it was expressed as inclusion bodies and thereby protected from degradation by cellular proteases.

### Example 2: Immunohistochemical studies using antiserum to 64TRP

The individual purified recombinant 64trp proteins were used to raise polyclonal antisera by subcutaneous injections of equal volumes of each protein and Montanide ISA 50 into Dunkin Hartley Guinea pigs.

Imnnunohistochemical studies were performed using the six different anti-64trp antisera. The antisera have been reacted with sections of both normal Hamster skin (from animals not exposed to ticks), and sections through cement cones and the surrounding skin of Hamsters infested with *R. appendiculatus* ticks. The immunohistochemical methods were as previously described (Coligan *et al.,* 1991).

The results of two of these studies, using anti-64trp2 and anti-64trp3 sera, indicate the following.

### 2.1 Normal skin

Comparison of normal skin sections treated with either anti-64trp2 or anti-64trp3 sera revealed that the anti-64trp3 serum reacted strongly with the epidermal and dermal tissues, particularly with the keratinocytes and cornified layer of the epidermis, and collagen fibres of the dermis (Figure 4A). By comparison, sections treated with the anti-64trp2 serum were indistinguishable from the control sections treated with phosphate buffered saline (PBS) (Figures 4B and 4C, respectively).

Both 64trp2 and 64trp3 protein sequences contain the collagen-like region of 64P protein. However, the 64trp3 protein also includes some of the keratin-like sequences of 64P protein. The differences in immunoreactivity between anti-64trp3 and anti-64trp2 antisera with the Hamster skin section are thus probably related to the availability of reactive epitopes within the respective protein sequence fragments. This further confirms the hypothesis that the 64P cement protein mimics the structure of certain of its host's tissues, e.g. collagen and especially the keratin-like proteins of the epidermis and dermis.

### 2.2 Tick-infested skin

In skin of Hamsters on which *R. appendiculatus* ticks fed, sections of individual cement cones reacted with anti-64trp3 serum. The reactions were mainly with the outermost layer and the inner layers of the cement cones attached to the epidermis (Figure 5A). No reactions were observed in sections treated with anti-64trp2 serum (Figure 5C), or with PBS used as a control instead of a primary antibody (Figure 5B).

The reaction pattern of anti-64trp3 serum with cement cones may indicate that the 64P is a cement protein that lines the cement cone, possibly acting as a glue that binds the cement cone to the surrounding epidermal and dermal tissues. The absence of staining within the cement cone may indicate that the epitope(s) recognised by the anti-64trp3 serum (and possibly the anti-64trp2 serum) were not exposed in the cement cone. This might occur if the 64P protein polymerises to form the cement cone.

As well as being embedded into the host's skin, the outer-most layer of the cement cone also tapers down and blends onto the epidermis of the host's skin (data not shown) such that it is difficult to distinguish where the tick cement cone ends and the host's tissue begins. This observation further supports the hypothesis that the cement protein has been designed to resemble the dermal and epidermal skin proteins (i.e. collagens and keratins) of the host, in order to avoid rejection of the tick via the attachment cement cone on the host's skin.

### Example 3: Vaccination trials

Dunkin-Hartley Guinea pigs were used for the immunisation and challenge trials. Of the 10 Guinea pigs used, 2 each were immunised with 64trp1, 64trp2, 64trp3 and 64trp6, and 2 were immunised with control protein GST. Each group of immunised Guinea pigs was further divided into one Guinea pig each per adult and nymphal feeding stages of *R*. *appendiculatus* ticks. Approximately fifty milligrams of each 64TRP protein (still attached to GST beads, i.e non-eluted) or control GST protein was mixed with ISA Montanide adjuvant and injected subcutaneously; first and second booster injections were each given at intervals of two weeks.

Host immune responses to the immunisation were determined by reactivity of 64TRP and GST immunised Guinea pig antisera on immunoblots of GST protein and individual 64TRP proteins.

When the antisera titres reached 1:5,000, the Guinea pigs were challenged with adult and nymphal stages of *R. appendiculatus* ticks. For the challenge infestation, pre-determined numbers of ticks (per Guinea pig, 50-200 nymphs or 20 adult ticks) were placed within retaining chambers on the back of each Guinea pig (Figure 7), one week after the second booster injection.

To analyse the effects of 64TRP-induced immunity, daily visual examinations were made starting 24h post-tick infestation of the Guinea pigs. Attachment rates, duration of feeding, inflammatory reactions at sites of attachment, and hatchability of eggs from detached replete female ticks were recorded. Adult female tick engorgement weights and mortality rates of both adult and nymphal stages of the tick were determined after termination of the experiment.

Table 1 summarises the results of the vaccine effects of 64TRP proteins against tick feeding in Guinea pigs. There were no differences in the attachment rates of the control GST-immunised group and the 64TRP-immunised groups; likewise, the duration of feeding was similar for both adult and nymphal stages of the tick.

Inflammatory reactions, which lasted 24-48h before subsiding, were observed during the late stages of feeding (6th-7th day) on the skin of 64TRP-immunised Guinea pigs at sites where nymphs were attached. The reactions included erythema, oedema, lymphadenopathy and lethargy (Figure 6).

**Table 1. Comparison of feeding parameters for Rhipicephalus appendiculatus ticks feeding on guinea pigs immunised with 64TRP proteins and GST protein.**

| | Immunised with GST | | Immunised with: | | | | | | | | | | | |
|---|---|---|---|---|---|---|---|---|---|---|---|---|---|---|
| Parameters | | | 64trp1 | | 64trp2 | | 64trp3 | | 64trp4 | | 64trp5 | | 64trp6 | |
| | A | N | A | N | A | N | A | N | A | N | A | N | A | N |
| Attachment (%) | 100 | 100 | 100 | 100 | 100 | 100 | 100 | 100 | 100 | 100 | 100 | 100 | 100 | 100 |
| Duration of feeding (days) | 7-10 | 5-10 | 7-11 | 5-10 | 7-11 | 5-10 | 7-11 | 5-9 | 7-10 | 5-9 | 7-9 | 5-10 | 7-10 | 5-10 |
| Inflammation of attachment site | - | - | - | + | - | ++ | - | ++ | - | + | - | + | - | +++ |
| Antibody Titer¹ (x10³) | >1:5 | >1:5 | 1:8 | >1:8 | 1:8 | 1:8 | >1:8 | >1:8 | 1:8 | 1:8 | >1:8 | >1:8 | 1:8 | 1:8 |
| Antibody Titer² (x10³) | 1:5 | 1:5 | 1:10 | 1:10 | >1:10 | >1:10 | >1:16 | >1:16 | 1:8 | 1:8 | 1:10 | >1:10 | 1:10 | 1:20 |
| Mean engorged wt. (mg) (±SD) | 447 (±107) | nd | 425 (±67) | nd | 336 (±175) | nd | 441 (±86) | nd | 329 (±141) | nd | 354 (±98) | nd | 498 (±92) | nd |
| ^{‡}Mean egg mass wt. (mg) (± SD) | 147 (± 27) | n/a | 145 (± 26) | n/a | 120 (± 102) | n/a | 163 (± 51) | n/a | 107 (± 48) | n/a | 83 (± 34) | n/a | 175 (± 30) | n/a |
| Egg hatchability | h | | h | | *h | | h | | h | | h | | h | |
| Mortality (%) | 0^{§} | 6.7^{δ} | 0^{§} | 11.5^{δ} | 55.5^{§} | 18^{δ} | 70^{§} | 1.9^{δ} | 0^{§} | 5.6^{δ} | 60^{§} | 5.4^{δ} | 0^{§} | 48.2^{δ} |
| Total no. ticks | 9 | 159 | 10 | 145 | 9 | 137 | 10 | 186 | 8 | 194 | 10 | 39 | 9 | 113 |

| | | | | | | | | | | | | | | |
|---|---|---|---|---|---|---|---|---|---|---|---|---|---|---|
| **Table 1 notes** *i*=Inflammation - and + denote absence or presence of inflammation at sites of tick attachment, respectively; degree of inflammation is indicated by: + =mild, ++ =moderate and +++ =severe; ¹ & ² = pre-and post-infestation antibody titres; nd=not done; n/a=not applicable; h=hatched; * refers to 2/9 batches of eggs that did not hatch, laid by 2 adult female ticks from the group of *R*. *appendiculatus* ticks that had fed on guinea-pigs immunised with 64trp2 protein; ^{δ}determined by Chi-Square Test using a one-way contingency table showing a higher mortality rate from the group of *R*. *appendiculatus* nymphal ticks that fed on 64trp6-immunised guinea pigs (χ²,_{6,} = 96.5, p<0.001); ^{§} determined by the G Test, mortality rates observed in adult female ticks from groups of *R. appendiculatus* ticks that had fed on guinea-pigs immunised with 64trp2, 64trp3 and 64trp5 proteins were significantly higher than for ticks from the other groups (*G,*_{*6*,} =33, p<0.001); ^{∈} determined by F ratio, mean engorgement weights for adult female ticks from the groups of *R. appendiculatus* ticks that had fed on guinea pigs immunised with 64trp2, 64trp4 and 64trp5 were significantly less than engorgement weights for ticks from the other groups (*F*,*_{1,62}* = 15.88, p<0.001); *^{‡}* determined by F ratio, showing a significantly lower mean egg mass weight (*F*,*_{1,44}* = 5.646, p<0.022) for female *R*. *appendiculatus* ticks that fed on 64trp5-immunised guinea pig as compared to female ticks from the other groups. | | | | | | | | | | | | | | |

### 3.1 Nymphal ticks

The detached nymphs were normal in appearance. Inflammation was not evident at nymphal attachment sites on the skin of GST-immunised Guinea pigs.

Detached nymphs were reared according to Jones et al., (1988) Animal Technology 39, 99-106. Comparatively high mortality rates of 18% and 48% were observed for the detached nymphs that had fed on 64trp2- and 64trp6-immunised Guinea pigs when compared to control protein-immunised Guinea pigs (6.7%). The results are statistically significant, p<0.01 and p<0.001, respectively, as determined by the Chi-Square Test using a 2 x 2 contingency table.

The deaths of nymphs fed on 64trp2- and 64trp6-immunised Guinea pigs suggest that an immune response was stimulated when the nymphal stage of *R*. *appendiculatus* ticks fed on 64TRP-immunised Guinea pigs. Although this response does not directly affect feeding it affects survival of the nymphs. It is possible that immuno-protective epitope(s) exists within 64trp2 protein (small N-terminal fragment of 64P protein) as well as within the 64trp6 protein (133 amino acid fragment of 64P protein, as denatured antigen), which may be conserved in a related cement protein in the nymphs. This will be investigated further by immunoblotting using anti-64trp antisera on nymphal tissue extracts.

### 3.2 Adult ticks

Mortality was also significant among adult female ticks fed on Guinea pigs immunised with 64trp2 (mortality 55.5%) and 64trp3 (mortality 70%) proteins. Figure 7A (no.1) shows some of the adult ticks that fed on the 64trp2-immunised Guinea pig. They had lower engorgement weights compared to the other 64trp- and GST- immunised Guinea pigs (Table 2; Mean weight = 336 mg). Furthermore, two ticks from this group laid small batches of eggs that did not hatch; and during the late stage of feeding, some of the replete detached adult ticks died 2 days after detachment.

Similarly, fully engorged, post-fed adult ticks from 64trp3-immunised Guinea pigs died within the first week after detachment (Figure 7B). These ticks were over distended with the blood meals compared to the control ticks, dark in colour and developed a hard consistency at death. This vaccine effect on the ticks indicates damage to the tick gut as well as a disturbance of osmoregulation that is a function of tick salivary glands.

All the normal adult ticks from the control protein-immunised Guinea pig appeared normal and survived to lay eggs (Figure 7C).

Necropsy studies were made of skin biopsies from Guinea pigs immunised with 64TRP proteins following feeding with *R*. *appendiculatus* adult ticks. Erythmatous papular lesions were observed at the tick attachment sites (Figures 8A, B and C, no.3) compared with skin biopsies from control protein-immunised Guinea pigs (Figures 8D and E).

The lesions are marked in the biopsy from 64trp2-immunised Guinea pig, indicated by thickening of the skin and necrotic lesions (Figure 8C).

These results correlate with high mortality observed in the adult ticks after feeding on 64TRP-immunised Guinea pigs. Thus tick feeding appears to have stimulated an immune response by the 64TRP-vaccinated Guinea pigs against the late stage of feeding of adult ticks.

To confirm these findings, histological studies were performed on sections from the skin biopsies using Haematoxylin and Eosin stains (Bancroft J. D., and Stevens, A., Eds. (1990). Theory and Practice of Histological Techniques. 3rd Edition). The results show the normal histological profile expected with the skin biopsy section from the control protein-immunised Guinea pig, post-feeding of adult ticks (Figure 9.1).

In contrast, histological studies of skin biopsy sections from 64TRP-immunised Guinea pigs confirm immunological responses to the feeding of adult ticks as seen by the presence of leucocytic infiltration particularly in the dermis close to previous tick attachment sites in the epidermis, which was observed at both low magnification (Figures 9.2 and 9.3) and high magnification (Figures 9.7 and 9.8). Epithelial hyperplasia is also present which is evidence of chronic inflammatory responses.

Further histological studies were performed on these same sections as well as the control sample using Hema "Gurr" Rapid Blood Smear stain (BDH) (i.e. Wright's stain), according to the manufacturer's recommendations. The results confirmed the leucocytic infiltrates to be eosinophil and basophil polymorphs in skin biopsy sections from 64trp-immunised Guinea pigs (Figures 9.5 and 9.6), which are not present in the control sample (Figure 9.4).

### 3.3 Egg_production and viability

Statistical analysis of egg production determined by F ratio showed a significantly lower mean egg mass weight (*F,_{1,44}* = 5.646, p<0.022) for female *R. appendiculatus* ticks that fed on 64trp5-immunised guinea pig as compared to female ticks from the other groups. In addition, 2/9 batches of eggs did not hatch. These were laid by 2 adult female ticks from the group of *R. appendiculatus* ticks that had fed on Guinea pigs immunised with 64trp2 protein. The results indicate that certain constructs have a significant effect on the reproductive output of adult female ticks fed on the immunised animals. This is a desirable vaccine effect.

The results from the vaccination trials support the use of 64trp2, 64trp3, 64trp5 and 64trp6 proteins as vaccines against adult and nymphal *R. appendiculatus* ticks.

The immune inflammatory responses observed are reminiscent of the immune responses detected with secondary tick infestation (Brown and Askenase, 1981 J. Immunol. 127: 2163-2167; Brown and Askenase, 1983 Federal Proceedings 42, 1744-1749) that included intense erythema, eodema, necrosis, hyperplasia and erythmatous papulae at abandoned tick feeding sites. The localised cell reaction (basophil and eosinophil polymorphs) and tissue reaction in secondary infestation, is known as cell-mediated immunity and is widely regarded as the primary mechanism of rejection in tick-immune animals. Hence, a complex immune mechanism exists involving a localised, cell-mediated reaction at the attachment site, as well as humoral and complement-dependent effector mechanisms.

The antibody titres were determined by ELISA using anti-64trp or anti-GST sera from guinea pigs immunised with 64trp or GST antigens, respectively (Desai et al., (1994) J. Neurol. Sci. 122, 109-116). Comparison of the titres before and after tick infestation showed consistent increases in antibody titres for Guinea pigs immunised with 64trp1, 64trp2, 64trp3, 64trp5, or 64trp6. The observed increases indicate that tick infestation of immunised animals has a booster effect, inducing an anamnestic response. This response indicates that natural tick infestation will obviate the need for further immunisations because tick feeding will provide the immune stimulus necessary to maintain vaccine protection. Thus a single-shot vaccine requiring only one immunisation can be prepared using an appropriate adjuvant and a single or cocktail of 64trp constructs.

### Summary of studies on 64P cement protein

The cement cone produced by *Rhipicephalus appendiculatus* acts as an anchor, securing the tick's mouthparts in the epidermis and dermis of its host's skin. The junction between the cement cone and the surrounding host tissue forms a leak-proof seal. To avoid provoking a host rejection response against the tick, the cement proteins have adopted a structure similar to that of collagen and keratin. Truncated forms of the cement protein (64P) has been expressed in *E. coli* and antisera raised to the proteins. A summary of the results is shown in Table 2 below.

**Table 2: Summary of the characteristics of 64TRP proteins**

| Property | 64P truncated protein: | | | | | |
|---|---|---|---|---|---|---|
| | 64trp1 | 64trp2 | 64trp3 | 64trp4 | 64trp5 | 64trp6 |
| 64P amino acid (aa) | 29 aa | C- 51 aa | N- 70 aa | N- 63 aa | C- 133 aa full- | 133 aa full- |
| sequence fragment | terminal clone | terminal clone | terminal clone | terminal clone | length clone | length clone |
| Molecular weight (kD) | 3 (30)* | 6 (33)* | 8 (36)* | 7 (34)* | 15 (41)φ | 15 (42)* |
| GST/HIS.TAG fusion protein | GST/ HIS.TAG | GST/ HIS.TAG | GST/ HIS.TAG | GST/ HIS.TAG | GST | GST/ HIS.TAG |
| Soluble/denatured protein | Soluble | Soluble | Soluble | Soluble | Soluble | Denatured |
| Vaccine effect | + | +++ | ++^{a} | - | ++^{a} | ++ⁿ |

| | | | | | | |
|---|---|---|---|---|---|---|
| Vaccine effect: + = mildly potent effect against *Rhipicephalus appendiculatus* nymphal ticks, post-feeding on 64trp1-immunised guinea pigs; +++ = very potent effect against *Rhipicephalus appendiculatus* nymphal and adult ticks, post-feeding on 64trp2-immunised guinea pigs; ++^{a} = very potent effect against *Rhipicephalus appendiculatus* adult ticks, post-feeding on 64trp3-immunised guinea pigs; ++ⁿ = very potent effect against *Rhipicephalus appendiculatus* nymph ticks, post-feeding on 64trp6-immunised guinea pigs; no effect against *Rhipicephalus appendiculatus* adult and nymphal ticks, post-feeding on 64trp4- immunised guinea pigs. * Molecular weight of fusion protein i.e. including 26 kD GST protein + 1 kD 9XHIS.TAG. φ Molecular weight of fusion protein i.e. including 26 kD GST protein. | | | | | | |

### Example 4: Cross-reactivity of antisera raised against Rhipicephalus appendiculatus cement protein 64 trp

### 4.1 Mechanism of action of tick cement vaccine

As set out above in Example 3, immunisation of guinea pigs with certain fragments of *R*. *appendiculatus* 64 trp resulted in high mortality in adult female and nymphal ticks after they had completed feeding. The ticks turned black and became rigid, suggesting damage to the ticks' gut and leakage of the bloodmeal from the gut into the body cavity.

To test this hypothesis, immunoblots have been carried out to determine whether antibodies directed against tick cement fragments cross-reacted with antigens in the gut and haemolymph of adult ticks, and with antigens in whole body extracts of nymphs and larvae.

### 4.2 Results

Anti-64 trp2 serum reacted with protein bands of 22 kD and 25 kD in cement cone extracts (Figure 10A). The antiserum showed cross-reactivity with protein bands in salivary glands (22, 25 kD), haemolymph (22, 52 kD), nymphal (52, 98 kD), and larval (52 kD) extracts (Figure 10A), and in midgut (52 kD) extracts (Figure 11B).

Anti-64 trp5 serum reacted with protein bands of 31 kD and 48 to 70 kD in cement cone extracts (Figure 11D). The antiserum cross-reacted with protein bands in salivary gland extracts of unfed ticks (15, 22 and 31; Figure 11C) and of ticks that had fed for 2 days (25, 31 kD; Figure 11D). Cross-reactivity was also detected with protein bands of midgut (52 to 70 kD; Figure 11C) and haemolymph (31, 48 kD; Figure 11D), but not with nymphal and larval extracts (Figure 11D). Similar cross-reactions were observed using anti-64 trp3 serum (not shown).

Anti-64 trp6 serum reacted with protein bands of 22, 25 kD and 48-70 kD in cement core extracts (Figure 10B). The antiserum cross-reacted with protein bands in salivary gland (22, 25 kD), haemolymph (22, 48 kD), and larval (120 kD) extracts (Figure 10B), and with midgut extracts (65 to 70 kD; Figure 10A), but showed no apparent cross-reaction with nymphal extracts (Figure 10B).

### 4.3 Conclusions

Antibodies raised against *R. appendiculatus* tick cement fragments of the keratin-like protein, 64trp, clearly cross-react with antigenic epitopes in the salivary gland, midgut and haemolymph of adult female *R. appendiculatus.* Reaction with these epitopes by antibodies in the bloodmeal of ticks fed on immunised animals presumably causes damage to the midgut resulting in death of the tick. Dissection of one of the affected female ticks revealed coagulated blood dispersed within the body cavity, consistent with rupture of the midgut. Thus although the vaccine comprises a secreted protein of ticks (i.e., 'exposed' antigens), it is causing high mortality by targeting 'concealed' antigens in the midgut and possibly also the haemolymph and salivary glands (i.e. affecting normal physiological functions). The cement fragments are therefore providing a dual action vaccine that:
(i) stimulates an inflammatory response which will boost the immune status of vaccinated animals, and
(ii) targets concealed antigens resulting in damage to the tick.

### Example 5: Cross-reactivity with other tick species using immunoblotting

To determine whether antibodies raised against *R. appendiculatus* cement protein react with antigenic epitopes of other tick species, immunoblotting was undertaken with tissue extracts of *Rhipicephalus sanguineus* (the dog tick), *Amblyomma variegatum* (an economically important pest of cattle in Africa, South America and the Caribbean), and *Ixodes ricinus* (the sheep or wood tick that transmits Lyme disease and tick-borne encephalitis to humans in Europe).

### 5.1 Results

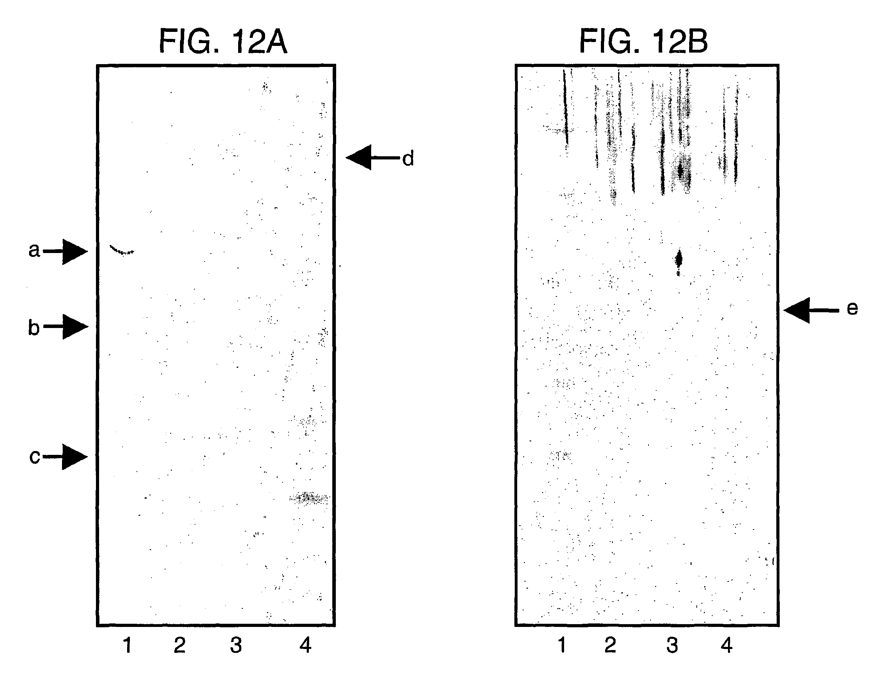

Anti-64 trp5 serum cross-reacted with protein bands of *A*. *variegatum* salivary gland (180 kD) and midgut (25, 52 kD) extracts and haemolymph (85 kD) (Figure 12A). There were no cross-reactions detected with *R. sanguineus* salivary glands, haemolymph or midgut (not shown).

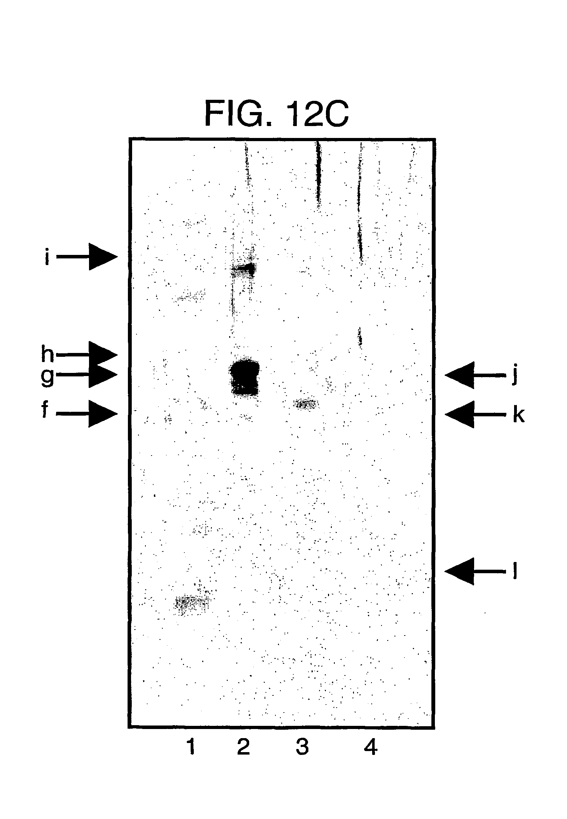

Anti-65trp6 serum cross-reacted with a 50 kD band of *A*. *variegatum* haemolymph but showed no activity with salivary gland and midgut preparations (Figure 12B). With *R*. *sanguineus,* cross-reactions occurred with several salivary gland protein bands (51, 53-55, 65, 120 kD) and with midgut (25, 52 and 55 kD) but not with haemolymph (Figure 12C). Cross-reacting bands that appeared fuzzy are probably glycosylated proteins.

Using 64trp3 antiserum (Figure 13A), two pronounced bands (a and b) and one faint band (c) were detected in nymphal extracts and there was also cross-reactivity with adult haemolymph, midgut and salivary glands.

Antiserum to 64trp2 (Figure 13B) detected several bands in *R. sanguineus* extracts, including one strong band (j) present in all extracts.

The control antiserum raised against GST detected cross-reacting bands in cement cone and salivary glands (Figure 14). The cross-reactions in salivary glands may represent *R*. *sanguineus* GST, as reported for *Boophilus microplus* and those in cement cones are probably due to non-specific reactions with host proteins haemoglobulin/IgG contaminating cement cone extracts that were obtained from partially fed ticks.

Antisera to 64trp2 and 64trp5 each cross-reacted with cement cone, gut, and whole nymphal extracts of *I*. *ricinus.* By contrast, no cross-reactions were detected with anti-64trp3. This observation differed from the cross-reactivity observed with tissue extracts of *R. sanguineus* and *A*. *variegatum.* There was also cross-reactivity of anti-64trp6 serum with gut and nymphs of *I*. *ricinus.*

The results are summarised in Table 3.

### 5.2 Conclusions

Antibodies raised to *R. appendiculatus* cement protein 64trp constructs cross-reacted with antigenic epitopes in the salivary glands, midgut and haemolymph of three other ixodid tick species. The results suggest that the candidate vaccine(s) derived from *R. appendiculatus* cement provide a broad spectrum vaccine that is effective against a number of different tick species.

On the basis of the observed cross-reactivities, 64trp6 of *R. appendiculatus* was selected as an immunogen for a vaccine trial. As demonstrated above, in vaccine trials with *R*. *appendiculatus* (see Table 2), 64trp6 was effective against *R. appendiculatus* nymphs but not against adults. Therefore, in vaccine trials with *R. sanguineus,* one of the two constructs (either 64trp3 or 64trp2) effective against adult *R. appendiculatus* was included with 64trp6, as described in the following section.

**Table 3: summary of results for cross-reactivity between R. appendiculatus, R. sanguineus, Amblyomma variegatum and Ixodes ricinus tick antigens using sera from guinea pigs immunised with 64trp recombinant antigens.**

| Antiserum | Tick Antigens | | | | | | | | | | | | | | | | | |
|---|---|---|---|---|---|---|---|---|---|---|---|---|---|---|---|---|---|---|
| | ***R. appendiculatus*** | | | | | | ***R. sanguineus*** | | | | | ***A. variegatum*** | | | | ***I. ricinus*** | | |
| | **CC** | **SG** | **gut** | **H** | **N** | **L** | **CC** | **SG** | **gut** | **H** | **N** | **SG** | **gut** | **H** | **CC** | **CC** | **gut** | **N** |
| **Anti-64trp2 ab' (50a.a. N-term. Frag. of 64P) Effective against RA adult/nymph ticks (soluble antigen)** | + | + | + | + | + | + | + | + | + | + | + | + | + | + | + | + | + | + |
| **Anti-64trp3 ab' (70a.a. N-term. Frag. of 64P) Effective against RA adult ticks (soluble antigen)** | + | + | + | + | + | + | + | + | + | + | + | + | + | + | + | - | - | - |
| **Anti-64trp5 ab' (133a.a. full- length clone of 64P) effective against RA adult ticks (soluble antigen)** | + | + | + | + | - | - | - | - | - | - | - | + | + | + | + | + | + | + |
| **Anti-64trp6 ab' (133a.a. full- length clone of 64P) effective against RA nymph ticks (denatured antigen)** | + | + | + | + | + | + | - | + | + | - | - | - | +^{∞} | + | + | - | + | + |
| **Anti-GST ab' control antiserum** | - | +^{g} | - | - | - | - | +* | +^{g} | - | - | - | +^{g} | - | - | -* | +* | +* | -* |

| | | | | | | | | | | | | | | | | | | |
|---|---|---|---|---|---|---|---|---|---|---|---|---|---|---|---|---|---|---|
| Codes used for Table 3 CC = tick cement cone extract, SG = salivary gland extract (from unfed ticks), gut = midgut extract, H =haemolymph, N = nymph tick extract, L =larval tick extract. + = positive and - = negative reactions respectively, to antisera used in immunoblots; ab' = antiserum; nd = not done. +* and +^{g} = positive reactions to anti-GST antiserum from insoluble fractions of CC and SG extracts solubilised at 100°C in SDS sample buffer. +* (CC from partially fed ticks) = immunopositive bands are probably due to non-specific binding of anti-GST ab' with host IgG/haemoglobulin fractions bound to cement cones. +*^{g}* = (SG from unfed ticks) = immuno-positive bands are probably due to specific binding of anti-GST ab' with an equivalent GST protein (Ref. *Boophilus microplus* GST protein). +^{∞} *A. variegatum* insoluble gut extract, solubilised in SDS/2-• mercaptoethanol sample buffer -* very faint immunopositive bands due to non-specific binding of nymph tick extract antigens with GST antiserum. | | | | | | | | | | | | | | | | | | |

### Example 6: Cross-protection vaccine trial: Guinea pigs immunised with Rhipicephalus appendiculatus cement protein 64 trp constructs and challenged with R. sanguineus adults.

### 6.1 Treatments for vaccine trial:

- Group 1: Recombinant 64trp6 + 64 trp2 + Montanide ISA (4 guinea pigs)
- Group 2: Recombinant 64 trp6 + 64trp3 + Montanide ISA (4 guinea pigs)
- Group 3: GST (control) (2 guinea pigs)

### • TOTAL NUMBER OF ANIMALS =10

### Route and dose:

Subcutaneous inoculation in the prescapular region either as combined antigens into a single site, or each antigen into different sites.

Dose: 50 µg antigen per animal.

### Vaccination scheme:

1. Primer inoculation
2. First boost
3. Test bleed at 10 to 12 days post-inoculation
4. Second boost (if antibody titre <1/5000)
5. Test bleed at 10 to 12 days post-inoculation
6. Antibody titre >1/5000: challenge with 10 pairs of *R. sanguineus* adults
7. Evaluate tick feeding performance, survival, reproductive output

### 6.2 Results

The results are summarised in Table 4. Overall, they show that putative vaccines derived against cement protein 64trp of *R. appendiculatus* were cross-protective against adults and nymphs of *R. sanguineus.* There were notable differences from previous observations with *R. appendiculatus.*

**Table 4. Comparison of feeding parameters for Rhipicephalus sanguineus ticks feeding on guinea pigs immunised with 64TRP proteins and GST protein.**

| Parameters | Immunised with GST-control protein | | | Immunised with: | | | | | | | | | | | |
|---|---|---|---|---|---|---|---|---|---|---|---|---|---|---|---|
| | | | | 64trp2/6: | | | | | | 64trp3/6: | | | | | |
| | | | | C* | | S* | | S* | C* | C* | | S* | | S* | C* |
| | M | F | N | M | F | M | F | N | N | M | F | M | F | N | N |
| Attachment (%) | 100 | 100 | 100 | 100 | 70 | 70 | 60 | 100 | 100 | 90 | 80 | 100 | 70 | 100 | 100 |
| Duration of feeding (days) | 10-13 | 10-13 | 5-8 | nd | 1-12 | nd | 1-10 | 5-8 | 5-8 | nd | 1-13 | nd | 1-13 | 5-8 | 5-8 |
| Inflammation of Attachment site¹ | - | - | - | ++ | ++ | ++ | ++ | + | + | +++ | +++ | ++ | ++ | + | + |
| Mean engorged wt. (mg) | nd | 207 | nd | nd | 195 | nd | 213 | nd | nd | nd | 203 | nd | 184 | nd | nd |
| Mortality (%) | 0 | 0 | 9.2 | 0 | 80 | 30 | 40 | 37 | 33 | 10 | 30 | 0 | 36 | 30 | 18.8 |
| Total no. ticks | 8 | 10 | 293 | 10 | 10 | 10 | 10 | 118 | 156 | 10 | 10 | 10 | 11 | 100 | 123 |
| Egg mass weight (mg)/ viability^{E} | 111 /E | | | 0^{†} | | 121 /E | | | | 14/E | | 106/E | | | |

| | | | | | | | | | | | | | | | |
|---|---|---|---|---|---|---|---|---|---|---|---|---|---|---|---|
| Table 4 notes * denotes guinea pigs individually immunised with 64trp protein cocktails (i.e. 64trp2/6 or 64trp3/6 combinations) either as combined antigens into single (C) or separate (S) subcutaneous sites. **I** refers to observed local inflammatory skin reactions at tick feeding sites (transient, 2-3 days) evident as erythema, oedema, scratching and lymphadenopathy. **nd** refers to not done. **M** and **F** refer to male and female adult ticks, respectively. **E** indicates eggs hatched. ^{†} indicates the two females that survived in this group did not lay eggs. | | | | | | | | | | | | | | | |

### (i) Effect on attachment

Unlike *R. appendiculatus,* the rate of attachment of *R. sanguineus* adults on test (but not control) animals was affected. At 24 hours post-infestation, most of the ticks on all 4 test animals were unattached and observed crawling on the retaining gauze as if attempting to leave the host. Subsequently, during the early period of feeding (day 1 to day 5), a total of 16/81 dead adults (12 females and 4 males) were removed from the test animals; all but one of these ticks appeared to be unfed (Figure 15). All 18 ticks on the control animal attached within 24 h of infestation.

### (ii) Effect on males

Again, unlike *R. appendiculatus,* an effect was observed on male survival. As mentioned above, 4 dead males were removed during the early feeding period.

### (iii) Inflammatory response

All 8 test guinea pigs showed an inflammatory response at the site of antigen inoculation and also in the area of tick feeding. Inflammation was first observed 6-7 days post-infestation, and had subsided by day 9-10 of feeding. No inflammation was observed on the control animals.

### (iv) Post-feeding mortality

As observed with *R. appendiculatus,* most of the females (29/41) completed engorgement. However, of these, 7 died within 2 days of detachment. They showed similar effects to *R*. *appendiculatus* (over-distension and blackening) consistent with damage or rupture to the gut (Figure 16). Moderate mortality rates where observed with nymphs from test animals, compared with the control case.

### (v) Reproductive output

Of the 22 surviving engorged females from the test animals, 19 laid eggs. Statistical analyses of these data are currently being determined.

### 6.3 Conclusions

1. Antibodies raised against the 64trp proteins cross-react in immunoblots with antigenic epitopes in the salivary gland and midgut of *R. sanguineus* ticks.
2. Cocktails of vaccines comprising different 64trp proteins constructed from a secreted cement protein (i.e. 'exposed' antigens) from the tick *R. appendiculatus,* provided cross-protection against another tick species *R. sanguineus* by targeting 'concealed' antigens in the midgut and salivary gland of adult ticks, causing high mortality.
3. The cocktail vaccines stimulate local inflammatory immune responses that will boost the immune status of vaccinated animals.

### Example 7: Antigenic cross-reactivity between Rhipicephalus appendiculatus and Boophilus microplus detected by immunoblotting using antisera to R. appendiculatus cement protein 64 trp constructs

### 7.1 Preparation of B. microplus tick extracts

*Boophilus microplus* adults and nymphs were collected from cattle. Note that the nymphs were fed and hence may show increased levels of non-specific cross-reactivity due to host proteins.

### 7.2 Results

### 7.2.1 Cross-reactivity with 64trp2 and 64trp3 antiserum

Cross-reactivity studies using immunoblotting with 64trp3 antiserum. (Figure 17A) detected several *B. microplus* cement cone proteins (**c**, **i**, **j**, **k**, and **l**); two bands of similar size (c and h) were detected in midgut and salivary glands. Many bands were detected in the fed nymphal extract, some of which were probably non-specific (see below).

Using 64trp2 antiserum (Figure 17B), one pronounced band (**o**) was detected in all extracts. This band is of similar size (62 kD) to band c detected in all samples using 64trp3 antiserum, although band **c** was not as pronounced. Band **c/o** was barely detectable in the Coomassie Blue stained gel (Figure 17C) indicating that cross-reactivity with 64trp2 or 64trp3 antiserum was likely to be specific and not due to non-specific binding. Also consistent with this interpretation is the fact that 64trp2 and 64trp3 proteins are related constructs with overlapping N-terminal sequences (see Figure 2).

With 64trp2 antiserum, a faint band (**q**) was detected in all samples. Cement cone (lane 5) and nymphal extract (lane 2) had common pronounced bands (**n** and **p**) while band r was detected in all adult extracts.

Immunopositive band designated as **e** are most likely due to non-specific binding of anti-GST antiserum with host proteins (haemoglobin/IgG) present in the tick tissue extracts. The immunopositive band **f** is probably due to specific binding of the anti-GST antiserum (see Figure 18C) with the protein bands in midgut, salivary gland and whole tick tissue extracts that represent the 26 kD GST protein of *Boophilus microplus* larvae (see He et al., (1999), Insect-Biochem-Mol-Biol. 29(80): 737-43).

### 7.2.2 Cross-reactivity with 64trp5 and 64trp6 antiserum

Both the 64trp5 antiserum and 64trp6 antiserum detected several bands in nymphal extracts but there was no obvious cross-reactivity with adult tick extracts (Figures 18A and 18B). The 64trp5 antiserum produced a dominant band (**b**) with cement cone but no cross-reactivity was detected between 64trp6 and cement cone extract.

Immunopositive bands designated as **a** and **e** are probably due to non-specific binding of anti-GST antiserum with host proteins (haemoglobin/IgG) present in cement cone extracts and whole nymph extract of fed ticks, respectively (Figure 18C). The immunopositive band f is probably due to specific binding of the anti-GST antiserum with the protein bands in midgut, salivary gland and whole tick tissue extracts, most likely the 26 kDa GST protein of *Boophilus microplus* larvae.

### 7.3 Conclusions

The results are summarised in Table 5.

Our vaccine trials with *R. appendiculatus, R. sanguineus* and *Ixodes ricinus* have shown that immunoblotting data provide a good indicator of effective vaccine immunogens. On this basis the results presented in this Example suggest that:
(i) 64trp2 and 64trp3 are candidate vaccine immunogens for controlling *B. microplus* adults and nymphs;
(ii) the 64trp5 construct may be effective against *B. microplus* nymphs and at least partially effective against adults;
(iii) 64trp6 may be effective against *B. microplus* nymphs and not adults;
(iv) cocktails of immunogens may be the most effective strategy for controlling *B*. *microplus:* either 64trp2 + 64trp5 or 64trp3 + 64trp5.

**Table 5: Cross-reactivity between R. appendiculatus and Boophilus microplus tick antigens using sera from guinea pigs immunised with 64trp recombinant antigens.**

| **Antiserum** | **Tick Antigens** | | | | | | | | | |
|---|---|---|---|---|---|---|---|---|---|---|
| | ***R. appendiculatus*** | | | | | | ***B. microplus*** | | | |
| | **CC** | **SG** | **gut** | **H** | **N** | **L** | **CC** | **SG** | **gut** | **N** |
| **Anti-64trp2 ab'. (50a.a. N-term. Frag. of 64P) effective against RA Adult/ nymph ticks (soluble antigen)** | **+** | **+** | **+** | **+** | **+** | **+** | **+** | **+** | **+** | **+** |
| **Anti-64trp3 ab' (70a.a. N-term. Frag. of 64P) effective against RS Adult/ nymph ticks (soluble antigen)** | **nd** | **nd** | **nd** | **nd** | **nd** | **nd** | **+** | **+** | **+** | **+** |
| **Antl-64trp5 ab' (133a.a. full- length clone of 64P) effective against RA adult ticks (soluble antigen)** | **+** | **+** | **+** | **+** | **-** | **-** | **+** | **-** | **-** | **+** |
| **Anti-64trp6 ab' (133a.a. full-length clone of 64P) effective against RA nymph ticks (de-natured antigen)** | **+** | **+** | **+** | **+** | **+** | **+** | **-** | **-** | **-** | **+** |
| **Anti-GST ab' control antiserum** | **-** | **-** | **-** | **-** | **-** | **-** | **+*** | **+*^{g}*** | **+*^{g}*** | **+*^{g}*/+*** |

| | | | | | | | | | | |
|---|---|---|---|---|---|---|---|---|---|---|
| CC =tick cement cone extract, SG =salivary gland extract, gut =midgut extract, H =haemolymph, N = nymph tick extract, L = larval tick extract. + =positive and - = negative reactions respectively, to antisera used in immunoblot. ab' =antiserum; nd =not done. +* and +*^{g}* = positive reactions to anti-GST antiserum from insoluble fractions of tick tissue extracts solubilised at 100°C in SDS sample buffer. +*(CC from partially fed ticks) immunopositive bands are probably due to non-specific binding of anti-GST ab' with host IgG/haemoglobulin fractions bound to cement cones and from whole nymphs, of fed ticks. +*^{g}* (SG from unfed ticks) immunopositive bands are probably due to specific binding of anti-GST ab' with an equivalent GST prolein¹. | | | | | | | | | | |

### Example 8: Cross-protection Vaccine Trial: Guinea pigs immunised with Rhipicephalus appendiculatus cement protein 64 trp constructs and challenged with I. ricinus nymphs

### 8.1 Selection of immunogens

A summary of the cement constructs of *R. appendiculatus* 64trp is shown in Figure 2. Candidate immunogens were identified on the basis of whether antiserum to the construct detected specific cross-reacting antigens in extracts of *I*. *ricinus* samples.

Cross-reactivity studies using immunoblotting with 64trp antisera showed detection of several *I ricinus* whole nymph and larval extract proteins (Figure 19), and adult preparations of cement cone and midgut extracts (not shown).

Using 64trp2 antiserum (Figure 19A(iii)), two major bands (**a** and **b**) were detected in nymphal extract and there was also cross-reactivity with adult cement cone and midgut extracts (see Table 3).

Antisera to 64 trp5 and 64trp6 (Figure 19A(iv) and (v), respectively) detected several bands in *I*. *ricinus* whole nymph extract. Antiserum to 64trp6 also detected 5 prominent bands in whole larva extract (Figure 19B(ii)).

The control antiserum raised against GST detected very faint bands in whole nymph, cement cone and midgut extracts which are probably due to non-specific reactions with host proteins haemoglobulin/IgG.

On the basis of the observed cross-reactivities, 64trp2, 64trp5 and 64trp6 of *R*. *appendiculatus* were selected as an immunogens for a vaccine trial. These were used singly or as cocktails.

### 8.2 Treatments for vaccine trial:

- Group 1: Recombinant 64trp6 + 64 trp2 + Montanide ISA (1 hamster)
- Group 2: Recombinant 64 trp6 + 64trp5 + Montanide ISA (1 hamster)
- Group 3: GST (control) (1 hamster)
- Group 4: Recombinant 64trp2 + Montanide ISA (1 hamster)
- Group 5: Recombinant 64trp5 + Montanide ISA (1 hamster)
- Group 6: Recombinant 64trp6 + Montanide ISA (1 hamster)

### Total Number of Animals = 6

### 8.3 Route and dose:

Subcutaneous inoculation in the prescapular region either singly or as combined antigens into a single site.

Dose 25 µg antigen per animal.

### 8.4 Vaccination scheme:

1. Primer inoculation
2. First boost
3. Test bleed at 10 to 12 days post-inoculation
4. Second boost (if antibody titre <1/5000)
5. Test bleed at 10 to 12 days post-inoculation
6. Antibody titre >1/5000: challenge with 50-100 *I. ricinus* nymphs
7. Evaluate tick feeding performance, local inflammatory immune response to tick feeding, survival and successive moulting to adults.

### 8.5 Results

The results are summarised in Table 6. Overall, they show that putative vaccines derived against cement of *R. appendiculatus* were cross-reactive against nymphs of *I. ricinus.*

### (i) Effect on attachment

Like *R. appendiculatus,* the rate of attachment of *I*. *ricinus* nymphs on test animals was not affected.

### (ii) Inflammatory response

Four hamsters showed an inflammatory response at the site of tick feeding with 3 hamsters having severe reactions (Figure 20). This result correlates with the *in vitro* assays (i.e. Western Blots). Inflammation was observed 3-5 days post-infestation, and was still present at tick detachment (day-5 of feeding). No inflammation was observed on the control animal.

### (iii) Post-feeding mortality

Cross-protection in relationship to *the in vitro* assays will be assessed when fed nymphs have moulted to adults.

### 8.6 Conclusions

1. Antibodies raised against the 64trp proteins cross-react in immunoblots with antigenic epitopes in nymphs, larvae and adult cement cones and midgut of *I*. *ricinus* ticks.
2. A host response was observed in hamsters immunised with 64trp immunogens (previous observations had only been in guinea pigs).
3. Various degrees of an inflammatory response were observed at the site of *I*. *ricinus* nymphal tick feeding on animals immunised with the 64 trp constructs, with the most severe response observed in a hamster immunised with a cocktail of 64 trp6/5.
4. The immunogens stimulate local inflammatory immune responses that will boost the immune response.

**Table 6. Comparison of feeding parameters for Ixodes ricinus ticks feeding on hamsters immunised with 64TRP proteins and GST protein.**

| Parameters | Immunised with GST | Immunised with: | | | | |
|---|---|---|---|---|---|---|
| | | 64trp2 | 64trp2/6 | 64trp5 | 64trp5/6 | 64trp6 |
| Attachment (%) | 100 | 100 | 100 | 100 | 100 | 100 |
| Duration of feeding (days) | 4-5* | 3-5 | 3-5 | 3-5 | 3-5 | 3-5 |
| Inflammation of attachment site^{I} | + | - | - | ++ | +++ | + |
| Mortality (%) | 18 | 34.9 | 63.6 | 34.2 | 29.3 | 25.7 |
| Total no. ticks | 149 | 63 | 36 | 73 | 143 | 136 |

| | | | | | | |
|---|---|---|---|---|---|---|
| *i*=Inflammation: - and + denote absence or presence of inflammation at sites of tick attachment during feeding, respectively; degree of inflammation is indicated by: + =mild, ++ =moderate and +++ =severe. * refers to tick feeding of nymphal *I*. ricinus ticks on control hamster, extended for 6 hours compared to those that fed on the 64trp-immunised hamsters. | | | | | | |

## Claims

1. A vaccine composition comprising a recombinant fragment of the 64P cement protein of the tick *Rhipicephalus appendiculatus* as an active component in conjunction with a pharmaceutically acceptable excipient, wherein said fragment is selected from the group consisting of:
64trp2 (an N-terminal fragment of the 64P tick cement protein consisting of 51 amino acids cloned as a glutathione-s-transferase and
histidine tag fusion protein wherein said 51 amino acids are amino acids 1-51 of the amino acid sequence in Figure 2);
64trp3 (an N-terminal fragment of the 64P tick cement protein consisting of 70 amino acids cloned as a glutathione-s-transferase and
histidine tag fusion protein wherein said 70 amino acids are amino acids 1-70 of the amino acid sequence in Figure 2);
64trp5 (a fragment of the 64P tick cement protein consisting of 133 amino acids cloned as a glutathione-s-transferase fusion protein wherein said 133 amino acids are amino acids 1-133 of the amino acid sequence in Figure 2);
64trp6 (a fragment of the 64P tick cement protein consisting of 133 amino acids cloned as a glutathione-s-transferase and histidine tag fusion protein wherein said 133 amino acids are amino acids 1-133 of the amino acid sequence in Figure 2);
and combinations thereof.

2. The composition according to claim 1 comprising the fragments 64trp2 and 64trp6.

3. The composition according to claim 1 comprising the fragments 64trp3 and 64trp6.

4. The composition according to any one of claims 1 to 3, additionally comprising an adjuvant.

5. A non-therapeutic and non-surgical method of production of an antibody or an antiserum that is reactive with a tick cement protein fragment as defined in claim 1, comprising immunising an animal with a vaccine composition according to any one of claims 1 to 4.

6. Use of a vaccine composition according to any one of claims 1 to 4 in the preparation of a medicament for immunising a mammal against a tick.

7. A vaccine composition according to any one of claims 1 to 4 for use in immunising a mammal against a tick.

## Patentansprüche

1. Impfstoffzusammensetzung, umfassend ein rekombinantes Fragment des Zementproteins 64P der Zecke *Rhipicephalus appendiculatus* als einen Wirkstoff in Verbindung mit einem pharmazeutisch verträglichen Exzipienten, wobei das Fragment ausgewählt ist aus der Gruppe bestehend aus:
64trp2 (ein N-terminales Fragment des 64P-Zecken-Zementproteins bestehend aus 51 Aminosäuren, die als ein Gluthathion-S-Transferase- und
Histidinmarker-Fusionsprotein cloniert sind, wobei die 51 Aminosäuren die Aminosäuren 1-51 der Aminosäuresequenz in Figur 2 sind);
64trp3 (ein N-terminales Fragment des 64P-Zecken-Zementproteins bestehend aus 70 Aminosäuren, die als ein Gluthathion-S-Transferase- und
Histidinmarker-Fusionsprotein cloniert sind, wobei die 70 Aminosäuren die Aminosäuren 1-70 der Aminosäuresequenz in Figur 2 sind);
64trp5 (ein Fragment des 64P-Zecken-Zementproteins bestehend aus 133 Aminosäuren, die als ein Gluthathion-S-Transferase-Fusionsprotein cloniert sind, wobei die 133 Aminosäuren die Aminosäuren 1-133 der Aminosäuresequenz in Figur 2 sind);
64trp6 (ein Fragment des 64P-Zecken-Zementproteins bestehend aus 133 Aminosäuren, die als ein Giuthathion-S-Transferase- und Histidinmarker-Fusionsprotein cloniert sind, wobei die 133 Aminosäuren die Aminosäuren 1-133 der Aminosäuresequenz in Figur 2 sind);
und Kombinationen davon.

2. Zusammensetzung gemäß Anspruch 1, umfassend die Fragmente 64trp2 und 64trp6.

3. Zusammensetzung gemäß Anspruch 1, umfassend die Fragmente 64trp3 und 64trp6.

4. Zusammensetzung gemäß einem der Ansprüche 1 bis 3, zusätzlich umfassend ein Adjuvans.

5. Nicht-therapeutisches und nicht-chirurgisches Verfahren zur Produktion eines Antikörpers oder eines Antiserums, welcher/welches mit einem wie in Anspruch 1 definierten Zecken-Zementprotein reaktiv ist, umfassend die Immunisierung eines Tieres mit einer Impfstoffzusammensetzung gemäß einem der Ansprüche 1 bis 4.

6. Verwendung einer Impfstoffzusammensetzung gemäß einem der Ansprüche 1 bis 4 für die Herstellung eines Medikamentes zur Immunisierung eines Säugetieres gegen Zecken.

7. Impfstoffzusammensetzung gemäß einem der Ansprüche 1 bis 4 für die Verwendung zur Immunisierung eines Säugetieres gegen Zecken.

## Revendications

1. Composition de vaccin, comprenant un fragment recombiné de la protéine 64P de cément de la tique *Rhipicephalus appendiculatus* en tant que composant actif avec un excipient pharmaceutiquement acceptable, ledit fragment étant choisi dans le groupe constitue par :
64trp2 (fragment N-terminal de la protéine 64P de cément de tique constitué de 51 acides aminées, cloné sous la forme d'une protéine de fusion contenant une glutathione-s-transférase et une étiquette d'histidine, lesdits 51 acides aminés étant les acides aminés 1 à 51 de la séquence d'acides aminés représentée sur la figure 2) ;
64trp3 (fragment N-terminal de la protéine 64P de cément de tique constitué de 70 acides aminés, cloné sous la forme d'une protéine de fusion contenant une glutathione-s-transférase et une étiquette d'histidine, lesdits 70 acides aminés étant les acides aminés 1 à 70 de la séquence d'acides aminés représentée sur la figure 2) ;
64trp5 (fragment de la protéine 64P de cément de tique constitué de 133 acides aminés, cloné sous la forme d'une protéine fusion contenant une glutathione-s-transférase, lesdits 133 acides aminés étant les acides aminés 1 à 133 de la séquence d'acides aminées représentée sur la figure 2) ;
64trp6 (fragment de la protéine 64P de cément de tique constitué de 133 acides aminés, cloné sous la forme d'une protéine fusion contenant une glutathione-s-transférase et une étiquette d'histidine, lesdits 133 acides aminées étant les acides aminés 1 à 133 de la séquence d'acides aminés représentée sur la figure 2) ;
et leurs combinaisons.

2. Composition selon la revendication 1, comprenant les fragments 64trp2 et 64trp6.

3. Composition selon la revendication 1, comprenant les fragments 64trp3 et 64trp6.

4. Composition selon l'une quelconque des revendications 1 à 3, comprenant en outre un adjuvant.

5. Procédé non thérapeutique et non chirurgical de production d'un anticorps ou d'un antisérum qui réagit avec un fragment de protéine de cément de tique selon la revendication 1, comprenant l'immunisation d'un animal avec une composition de vaccin selon l'une quelconque des revendications 1 à 4.

6. Utilisation d'une composition de vaccin selon l'une quelconque des revendications 1 à 4, dans la préparation d'un médicament destiné à immuniser un mammifère contre une tique.

7. Composition de vaccin selon l'une quelconque des revendications 1 à 4, utilisable pour immuniser un mammifère contre une tique.
